# EUROPEAN PATENT APPLICATION

(11) **EP 4 447 063 A1**
(43) Date of publication of application: **16.10.2024**
(21) Application number: 24169337.3
(22) Date of filing: 09.04.2024
(51) Int. Cl.: G16H 10/60, G16H 40/20, G16H 50/20

(54) **ABNORMALITY DETECTION SYSTEM AND BASE STATION DEVICE**

(30) Priority: 14.04.2023 JP 2023066750
(71) Applicant: Suzuki Motor Corporation, Shizuoka 432-8611 (JP)
(72) Inventor: TAKAGAITO, Fumiya, Shizuoka, 432-8611 (JP); KAMIYA, Naoki, Shizuoka, 432-8611 (JP); NAGAE, Yusuke, Shizuoka, 432-8611 (JP)
(74) Representative: Haseltine Lake Kempner LLP

(57) **Abstract**

Abnormal driving of the user in the vehicle is detected from vehicle data D1 including traveling information D13 of the vehicle, and medical information created based on information related to the detected abnormal driving of the user and an opinion of a medical worker of the medical institution is notified to a user terminal.

## Description

### TECHNICAL FIELD

The present invention relates to an abnormality detection system and a base station device.

### BACKGROUND ART

For example, Patent Literature 1 discloses a dementia risk determination system in which a situation determination unit determines a driving situation of a vehicle based on road information acquired by a road information acquisition unit and traveling information of the vehicle acquired by a vehicle information acquisition unit, and a violation determination unit determines whether the driving situation corresponds to a predetermined traffic violation that is likely to be performed when a cognitive function deteriorates. In the dementia risk determination system, when the driving situation of the vehicle corresponds to the predetermined traffic violation, a risk determination unit determines whether there is a dementia risk based on information related to a driver of the vehicle and a violation history obtained by a driver information detection unit. When the risk determination unit determines that there is a dementia risk, an output unit outputs the information.

### CITATION LIST

### PATENT LITERATURE

Patent Literature 1: JP2019-124975A

### SUMMARY OF INVENTION

### TECHNICAL PROBLEM

A cognitive function inspection is currently conducted at various facilities to confirm a cognitive function state of an elderly person whose risk of deterioration in the cognitive function increases with age. For example, when renewing a driver's license for the elderly person, the cognitive function inspection is conducted to measure memory and judgment using an inspection form, an electronic terminal, and the like. Such a cognitive function inspection is not equivalent to a medical inspection conducted by a medical institution, but is merely a simple inspection to confirm a state of memory and judgment of an examinee. Although these cognitive function inspections can confirm the cognitive function state at the time of the inspection, the cognitive function inspections cannot confirm the cognitive function state in daily life, such as when actually driving. Therefore, it is difficult to detect an abnormality of the cognitive function in an initial stage in which an indication or a symptom of deterioration in cognitive function which the user is not aware of is not always observed. From such a viewpoint, it is desired to discover an indication or a symptom of a disease related to a cognitive function of a user at an early stage from daily behavior such as driving of a vehicle.

The present invention has been made in view of the above circumstances, and an object thereof is to provide a base station device and an abnormality detection system capable of quickly discovering an indication or a symptom of a disease related to a cognitive function of a user by providing, to a medical institution, information related to a driving situation of the user in a vehicle.

### SOLUTION TO PROBLEM

In order to achieve the above object, an abnormality detection system according to the present invention includes: a service providing device including a detection unit configured to detect abnormal driving of a user of a vehicle from vehicle data including at least traveling information of the vehicle, and a notification unit configured to issue a notification related to medical information to the user; and a user terminal configured to communicate with the service providing device, in which the notification unit notifies the user terminal of medical information created based on information related to the abnormal driving detected by the detection unit and an opinion of a medical worker of a medical institution.

In order to achieve the above object, an abnormality detection system according to the present invention includes: a service providing device including a storage unit configured to store user data related to a user of a vehicle and vehicle data including traveling information of the vehicle, a detection unit configured to detect abnormal driving of the user in the vehicle from the vehicle data and store abnormality detection data including a content of the detected abnormal driving in the storage unit, and a notification unit configured to issue a notification related to medical information to the user; and a user terminal configured to communicate with the service providing device, in which the notification unit transmits at least the abnormality detection data to a medical institution terminal of a medical institution, receives, from the medical institution terminal, medical information data created based on the abnormality detection data and an opinion of a medical worker of the medical institution, and notifies the user terminal of the medical information including a content based on the medical information data.

In order to achieve the above object, a base station device according to the present invention includes: a service providing device including a storage unit configured to store user data related to a user of a vehicle and vehicle data including traveling information of the vehicle, a detection unit configured to detect abnormal driving of the user in the vehicle from the vehicle data and store abnormality detection data including a content of the detected abnormal driving in the storage unit, and a notification unit configured to issue a notification related to medical information to the user, in which the notification unit transmits at least the abnormality detection data to a medical institution terminal of a medical institution, receives, from the medical institution terminal, medical information data created based on the abnormality detection data and an opinion of a medical worker of the medical institution, and notifies the user terminal of the medical information including a content based on the medical information data.

### ADVANTAGEOUS EFFECTS OF INVENTION

An abnormality detection system and a base station device according to the present invention provide information related to a driving situation of a user in a vehicle to a medical institution, thereby exerting an effect of being able to discover an indication or a symptom of a disease related to a cognitive function of the user at an early stage.

### BRIEF DESCRIPTION OF DRAWINGS

[FIG. 1] FIG. 1 is a block diagram showing a schematic configuration of an abnormality detection system according to a first embodiment.
[FIG. 2] FIG. 2 is a block diagram showing a schematic configuration of the abnormality detection system according to the first embodiment.
[FIG. 3] FIG. 3 is a schematic diagram illustrating main data treated by the abnormality detection system according to the first embodiment.
[FIG. 4] FIG. 4 is a block diagram showing a schematic configuration of an in-vehicle device included in the abnormality detection system according to the first embodiment.
[FIG. 5] FIG. 5 is a block diagram showing a schematic configuration of a data processing device included in the abnormality detection system according to the first embodiment.
[FIG. 6] FIG. 6 is a block diagram showing a schematic configuration of an integration server included in the abnormality detection system according to the first embodiment.
[FIG. 7] FIG. 7 is a schematic diagram showing an example of a database stored in the integration server included in the abnormality detection system according to the first embodiment.
[FIG. 8] FIG. 8 is a block diagram showing a schematic configuration of a detection device included in the abnormality detection system according to the first embodiment.
[FIG. 9] FIG. 9 is a block diagram showing a schematic configuration of a user terminal included in the abnormality detection system according to the first embodiment.
[FIG. 10] FIG. 10 is a diagram showing an example of a notification of a consultation recommendation displayed on the user terminal included in the abnormality detection system according to the embodiment.
[FIG. 11] FIG. 11 is a diagram showing an example of the notification of the consultation recommendation displayed on the user terminal included in the abnormality detection system according to the embodiment.
[FIG. 12] FIG. 12 is a diagram showing an example of the notification of the consultation recommendation displayed on the user terminal included in the abnormality detection system according to the embodiment.
[FIG. 13] FIG. 13 is a block diagram showing a schematic configuration of a medical institution terminal included in the abnormality detection system according to the first embodiment.
[FIG. 14] FIG. 14 is a diagram showing an example of a flow (sequence) of a process performed by each processing unit of the abnormality detection system according to the first embodiment.
[FIG. 15] FIG. 15 is a diagram showing an example of a flow (sequence) of a process performed by each processing unit of the abnormality detection system according to the first embodiment.
[FIG. 16] FIG. 16 is a block diagram showing a schematic configuration of an abnormality detection system according to a second embodiment.
[FIG. 17] FIG. 17 is a block diagram showing a schematic configuration of the abnormality detection system according to the second embodiment.
[FIG. 18] FIG. 18 is a block diagram showing a schematic configuration of a mobility server included in the abnormality detection system according to the second embodiment.
[FIG. 19] FIG. 19 is a block diagram showing a schematic configuration of a medical server included in the abnormality detection system according to the second embodiment.
[FIG. 20] FIG. 20 is a diagram showing an example of a flow (sequence) of a process performed by each processing unit of the abnormality detection system according to the second embodiment.
[FIG. 21] FIG. 21 is a diagram showing an example of a flow (sequence) of a process performed by each processing unit of the abnormality detection system according to the second embodiment.
[FIG. 22] FIG. 22 is a block diagram showing a schematic configuration of an abnormality detection system according to a third embodiment.
[FIG. 23] FIG. 23 is a block diagram showing a schematic configuration of the abnormality detection system according to the third embodiment.
[FIG. 24] FIG. 24 is a block diagram showing a schematic configuration of a mobility server included in the abnormality detection system according to the third embodiment.
[FIG. 25] FIG. 25 is a block diagram showing a schematic configuration of an analysis device included in the abnormality detection system according to the third embodiment.
[FIG. 26] FIG. 26 is a block diagram showing a schematic configuration of a medical server included in the abnormality detection system according to the third embodiment.
[FIG. 27] FIG. 27 is a diagram showing an example of a flow (sequence) of a process performed by each processing unit of the abnormality detection system according to the third embodiment.
[FIG. 28] FIG. 28 is a diagram showing an example of a flow (sequence) of a process performed by each processing unit of the abnormality detection system according to the third embodiment.

### DESCRIPTION OF EMBODIMENTS

Hereinafter, embodiments according to the present invention will be described in detail with reference to the drawings. The present invention is not limited to these embodiments. In addition, the constituent elements in the following embodiments include components easily replaced by a person skilled in the art or essentially the same components.

### [First Embodiment]

### <Overview of Abnormality Detection System>

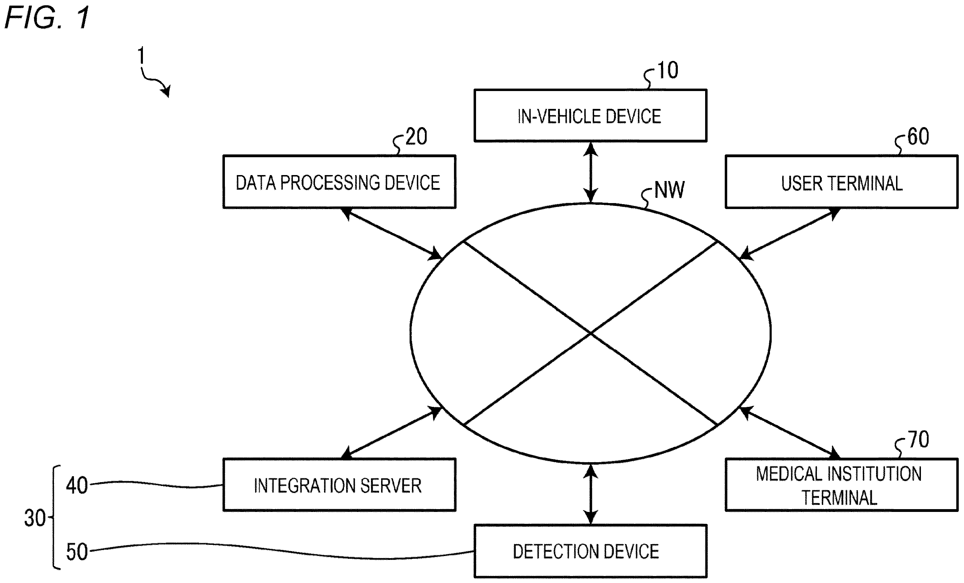

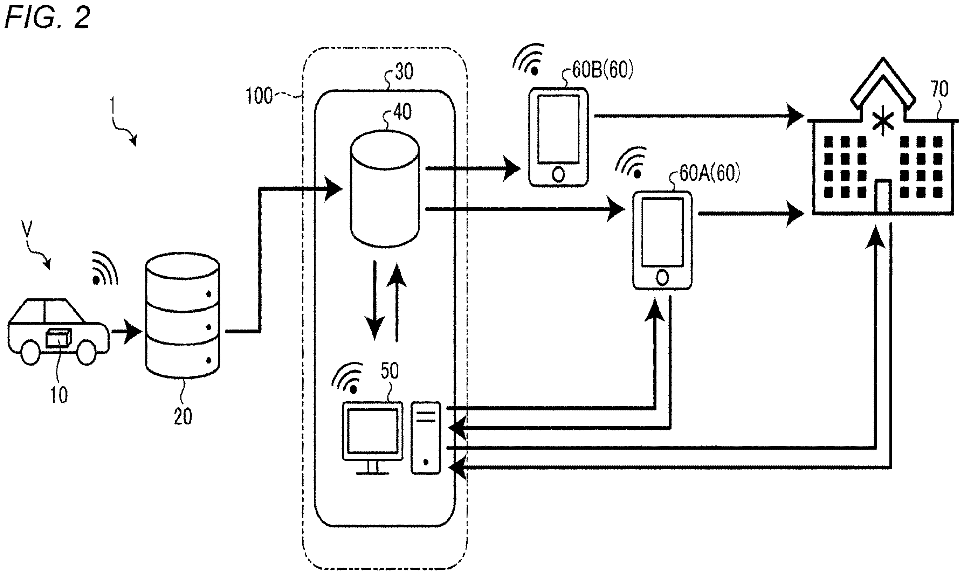

An abnormality detection system 1 according to a first embodiment shown in FIGS. 1 and 2 includes an in-vehicle device 10, a data processing device 20, a service providing device 30, a user terminal 60, and a medical institution terminal 70, and these devices and terminals are configured to communicate with each other via a network NW. The network NW constitutes a communication network that connects a plurality of devices so as to be able to communicate with each other, and enables data communication by various communication methods that can be applied to the present system regardless of wireless communication or wired communication. The network NW may include, for example, an Internet line network, a mobile phone line network, a mobile communication network, a local area network (LAN), a wide area network (WAN), and a virtual private network (VPN), and various network relay devices such as an antenna, a gateway, a router, and a hub may be interposed. The abnormality detection system 1 according to the present embodiment constitutes a system in which the in-vehicle device 10, the data processing device 20, the service providing device 30, the user terminal 60, and the medical institution terminal 70 can perform data communication with each other via the network NW to cooperate with each other to manage various kinds of data related to a vehicle V, and the data can be used for various services and analyses.

In such an abnormality detection system 1, the service providing device 30 according to the present embodiment includes an integration server 40 and a detection device 50, and an efficiency of utilization of data is improved by collectively storing, in the integration server 40, various kinds of data treated by the abnormality detection system 1. The abnormality detection system 1 according to the present embodiment detects abnormal driving of a user who is a driver of the vehicle V from a daily driving behavior of the user, and if the abnormal driving of the user in the vehicle V is detected, the abnormality detection system 1 provides abnormality detection data including information related to the abnormal driving to a medical institution. In the abnormality detection system 1 according to the present embodiment, the service providing device 30 receives medical information data created based on the abnormality detection data provided to the medical institution and an opinion of a medical worker such as a doctor in the medical institution, and the user is notified of the medical information including a content based on the medical information data.

The abnormality detection system 1 detects the driving behaviors, such as sudden braking, sudden steering, and overlooking of a temporary stop sign, as abnormal driving of the user of the vehicle V The abnormality detection system 1 according to the present embodiment may detect, as the abnormal driving of the user, a matter that sudden braking, sudden steering, overlooking of a temporary stop sign, and the like occur a predetermined number of times or more within a predetermined period (for example, within a period where the vehicle travels 100 km), for example.

In the abnormality detection system 1 according to the present embodiment, data stored in the integration server 40 is utilized by the detection device 50. The detection device 50 detects abnormal driving of the user based on data stored in the integration server 40. The detection device 50 provides the medical institution with information related to a content of the abnormal driving of the user whose abnormal driving is detected and the like as abnormality detection data D4. The abnormality detection data D4 is provided to the medical institution by the detection device 50 transmitting the abnormality detection data D4 to the medical institution terminal 70 provided at the medical institution. In the abnormality detection system 1 according to the present embodiment, the integration server 40 constitutes a storage unit that stores the vehicle data D1 including traveling information D13 of the vehicle V, user data D2 related to the user of the vehicle V, and the abnormality detection data D4. The detection device 50 includes a detection unit that detects abnormal driving of the user in the vehicle V from the vehicle data D1 and stores the abnormality detection data D4 including information related to the content or the like of the detected abnormal driving in the storage unit (the integration server 40), and a notification unit that transmits at least the abnormality detection data D4 to the medical institution terminal 70 of the medical institution, receives, from the medical institution terminal 70, medical information data D5 created based on the abnormality detection data D4 and an opinion of a medical worker in the medical institution, and notifies the user terminal 60 of medical information including a content based on the medical information data D5. In this case, the notification unit may be configured to transmit the abnormality detection data D4 and the user data D2 in association with each other to the medical institution terminal provided at the medical institution when the detection unit detects the abnormal driving of the user in the vehicle V

In the present embodiment, the medical institution terminal 70 is typically a terminal device installed at a medical institution such as a university hospital or a general hospital. The medical institution terminal 70 receives the abnormality detection data D4 transmitted from the detection device 50. The medical institution terminal 70 can process information related to the content of the abnormal driving included in the abnormality detection data D4 into a state of being available for a doctor or the like of the medical institution, and display the information on a display unit or the like. For example, the abnormality detection data D4 includes information related to the number of times of sudden braking, sudden steering, and overlooking of a temporary stop sign within a predetermined period (for example, within a period where the vehicle V travels 100 km), a surrounding situation in a case where the sudden braking, sudden steering, and overlooking of a temporary stop sign occurs, a state of the user captured by an in-vehicle camera, and biological information of the user.

From researches related to a relation between a cognitive function of a driver and a driving behavior, the present inventor has found that a correlation relation is present between and a cognitive function state of the driver and specific driving behaviors, such as sudden braking, sudden steering, and overlooking of a temporary stop sign. Specifically, the present inventor has found that, compared to a case where a person having a normal cognitive function drives the vehicle V, in a case where a person having an abnormal cognitive function drives the vehicle V, sudden braking, sudden steering, and overlooking of a temporary stop sign tends to increase. The present inventor has found that, compared to the case where the person having the normal cognitive function drives the vehicle V, in the case where the person having the abnormal cognitive function drives the vehicle V, an inter-vehicle distance between the vehicle V of the user and a vehicle traveling in front of the vehicle V of the user tends to be shorter than an appropriate inter-vehicle distance. The present inventor considers that the number of times of specific driving behaviors, such as sudden braking, sudden steering, and overlooking of a temporary stop sign, within a predetermined period (for example, within a period where the vehicle V travels 100 km) and the inter-vehicle distance to a preceding vehicle are utilized as one of determination materials for determining presence or absence of an indication or a symptom of a disease related to the cognitive function of the user in the vehicle V In the present embodiment, the abnormality detection data D4 transmitted from the detection device 50 to the medical institution terminal 70 is utilized, and the medical worker such as a doctor of the medical institution determines presence or absence of an indication and a symptom of a disease related to the cognitive function of the user based on an opinion of the user himself or herself or a reference created based on a correlation relation between the driving operation of the user in the vehicle V and the cognitive function state of the user.

Specifically, for example, the medical worker such as a doctor of the medical institution confirms, from the abnormality detection data D4, an abnormal driving operation of the user, such as sudden braking, sudden steering, and overlooking of a temporary stop sign, and a surrounding situation of the vehicle V during the abnormal driving operation, and identifies the abnormal driving operation that cannot be determined to be a normal driving operation. The doctor determines the presence or absence of the indication and the symptom of the disease related to the cognitive function of the user based on the number of times of each type of the abnormal driving operation that cannot be determined to be the normal driving operation (number of times of sudden braking, sudden steering, overlooking of a temporary stop sign, and the like), the reference created based on the correlation relation between the driving operation of the user in the vehicle V and the cognitive function state of the user, the state of the user captured by the in-vehicle camera during driving, and an opinion of the doctor himself or herself. Then, the doctor of the medical institution creates the medical information data D5 based on the determination result and stores the medical information data D5 in the storage unit of the medical institution terminal 70. For example, if the doctor of the medical institution determines that there is an indication or a symptom of a disease related to a cognitive function in the user of vehicle V, the doctor may store, as the medical information data D5 in the storage unit of the medical institution terminal 70, information related to a treatment program determined to be suitable for the user by the doctor of the medical institution and a consultation recommendation for the disease related to the cognitive function, together with the information related to a determination result related to the cognitive function of the user. In this way, the medical information data D5 includes information related to at least any one of the determination result related to the cognitive function of the user, a treatment program determined to be suitable for the user by the doctor of the medical institution, and the consultation recommendation on the disease related to the cognitive function.

The medical information data D5 stored in a storage unit of the medical institution terminal 70 is transmitted to the service providing device 30 by the medical institution terminal 70. The transmission of the medical information data D5 from the medical institution terminal 70 to the service providing device 30 may be automatically performed every time the medical information data D5 is stored in the medical institution terminal 70, or may be automatically performed at a constant cycle (for example, every several hours). The transmission of the medical information data D5 from the medical institution terminal 70 to the service providing device 30 may be manually performed by the doctor in the medical institution or a person in charge other than the doctor.

The medical information data D5 includes, as the determination result related to the cognitive function of the user, the information related to the presence or absence of the indication or the symptom of the disease related to the cognitive function of the user determined by the medical worker such as a doctor of the medical institution. The medical information data D5 includes, as a treatment program, information related to therapy determined to be suitable for the user by the medical worker such as a doctor. For example, the medical worker such as a doctor selects therapy considered to be suitable for the user from medication therapy, occupational therapy such as exercise therapy, reminiscence therapy, music therapy, and cognitive stimulation therapy, and the like, and stores, in the storage unit of the medical institution terminal 70, information related to therapy included in the medical information data D5 as a treatment program. The medical information data D5 includes information related to a consultation recommendation to the medical institution or a consultation recommendation on a cognitive function inspection as a consultation recommendation on the disease related to the cognitive function. The consultation recommendation on the disease related to the cognitive function is, for example, information related to the consultation recommendation to a specific medical institution determined to be suitable for the user by the medical worker such as a doctor. The selection of the medical institution in the consultation recommendation is not limited to an introduction by the doctor, and the detection device 50 may select the medical institution from the user data D2 or the like on the basis of a residence, a medical history, or the like of the user, add the selected medical institution to a medical information notification, and transmit the medical information notification to the user terminal 60.

In response to receiving the medical information data D5 from the medical institution terminal 70, the detection device 50 issues a medical information notification including the content based on the medical information data D5 to the user. In the present embodiment, the user terminal 60 typically includes a first user terminal 60A owned by the user himself/herself and a second user terminal 60B owned by a family of the user, a related person, or the like.

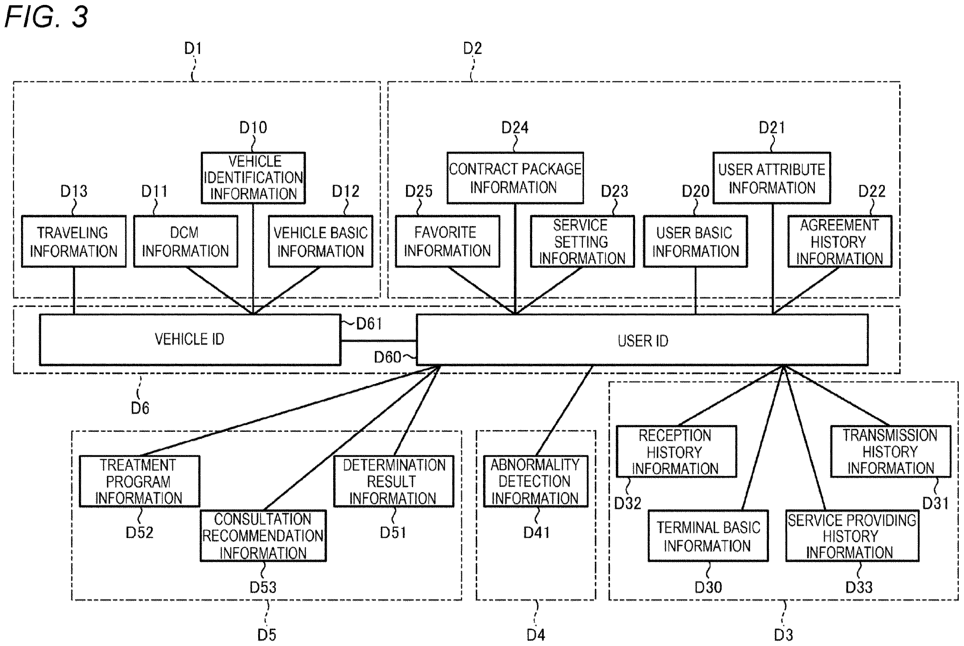

Hereinafter, configurations of the abnormality detection system 1 will be described in detail with reference to the drawings. In the following, first, an overview of main data treated by the abnormality detection system 1 will be described with reference to FIG. 3, and thereafter, the configurations of the abnormality detection system 1 will be described in detail with reference to other drawings and the like.

In the abnormality detection system 1, the vehicle V to be treated may be any driving system such as a gasoline vehicle, a diesel vehicle, a hybrid electric vehicle (HEV), a plug-in hybrid electric vehicle (PHEV), or an electric vehicle (EV). A driving method of the vehicle V may be any of manual driving, semi-automatic driving, fully automatic driving, and the like. In addition to an own vehicle of the user who can enjoy a service provided by the abnormality detection system 1, the vehicle V may be any one of a truck, a bus, a taxi, a special vehicle, and the like that can be identified as being used by the user.

### <Main Data Treated by Abnormality Detection System>

The main data treated by the abnormality detection system 1 includes, for example, the vehicle data D1, the user data D2, terminal data D3, abnormality detection data D4, the medical information data D5, and ID data D6 as shown in FIG. 3. The vehicle data D1, the user data D2, the terminal data D3, and the abnormality detection data D4, and the medical information data D5 are data-managed in association with the ID data D6 and the like in the abnormality detection system 1.

### <Vehicle Data>

The vehicle data D1 is data related to the vehicle V Typically, the vehicle data D1 is data collected by the in-vehicle device 10 mounted on the vehicle V and transmitted to a service providing device 30 side. Here, the vehicle data D1 includes, for example, data related to vehicle identification information D10, DCM information D11, vehicle basic information D12, and traveling information D13.

The vehicle identification information D10 is unique information assigned to each vehicle V to identify the vehicle V, and is, for example, information such as a vehicle identification number (VIN). In addition, the vehicle identification information D10 may be information such as a vehicle registration number (so-called car number) or a frame number of a vehicle. The DCM information D11 is information such as a type or an international mobile equipment identifier (IMEI) of a data communication module (DCM) mounted on the vehicle V The vehicle basic information D12 is information such as a vehicle name, a vehicle type, a model, and a grade of the vehicle V The traveling information D13 is information related to a traveling situation of the vehicle V In the present embodiment, the traveling information D13 includes driving information, vehicle state information, vehicle position information, device operation information, and image information.

The driving information is basic information related to driving of the vehicle V, and is, for example, information such as a vehicle speed, acceleration/deceleration, fuel consumption, an accumulated traveling distance, and a battery remaining amount. The driving information may include information related to the weather during traveling or information related to sound in the vehicle during traveling. The vehicle state information is detailed information related to a state of the vehicle V, and is, for example, information such as a steering wheel steering angle, an engine coolant temperature, an accelerator opening degree (an accelerator operation amount), a brake opening degree (an accelerator operation amount), an engine rotation speed, an engine torque, a motor torque, and a motor rotation speed. The vehicle position information is information such as position information (GNSS information, GPS information) of the vehicle V The device operation information is information related to an operation state of a device mounted on the vehicle V, and is, for example, information such as a door lock opening and closing state, a hazard lamp lighting state, a direction indicator operation state, a vehicle warning lamp lighting state, and presence or absence of an emergency notification. The image information is, for example, information such as a moving image inside and outside the vehicle captured in the vehicle V In addition, for example, information such as an acquisition date and time of information is added to the vehicle data D1. In addition to the above, the vehicle data D1 may include, for example, data related to the biological information of the user (driver) of the vehicle V The biological information of the user may include, in addition to various kinds of physiological information issued by a living body (user), various kinds of information derived from the physiological information, and is, for example, vital sign information such as a heart rate, a respiratory rate, a pulse rate, a blood pressure, and a body temperature, biological identification information such as a fingerprint, a voiceprint, and an iris, and information such as a blood alcohol concentration and an arousal level.

### <User Data>

The user data D2 is data related to the user of the vehicle V Here, the user in this abnormality detection system 1 is the driver of the vehicle V, and the abnormality detection system 1 detects abnormal driving, and if the abnormal driving is detected, the user receives the medical information notification based on the determination of the doctor of the medical institution. A service utilizer who enjoys a service provided by the abnormality detection system 1 or a contractor who makes a contract for receiving the service is also included in the user who uses the abnormality detection system 1. That is, the user data D2 can also be referred to as contractor data related to the service or customer data for a business operator who develops the service. Typically, the user data D2 is data registered in the integration server 40 by the in-vehicle device 10, the user terminal 60, or the like, or data automatically generated on an integration server 40 side based on the data registered in the integration server 40. Here, the user data D2 includes, for example, data related to user basic information D20, user attribute information D21, agreement history information D22, service setting information D23, contract package information D24, favorite information D25, and the like.

The user basic information D20 is information related to the contractor (customer) registered by the user himself or herself, and is, for example, information such as a user name, a postal code, an address, a mail address, a telephone number, a work contact address, a personal number (my number) of the user, and an insurer number. Here, the personal number (my number) is an identification number of an individual and is a number given to a person having a resident card in Japan based on the law in Japan. The insurer number is a number assigned to an insurer of a medical insurance for which the user insures, and is a number capable of identifying the insurer.

The user attribute information D21 is information related to an attribute of the user registered by the user himself or herself or automatically generated on the integration server 40 side, and is, for example, information such as age, gender, occupation, and nationality. The agreement history information D22 is information related to various agreement histories automatically generated on the integration server 40 side, and is, for example, information related to the presence or absence of agreement with respect to a privacy policy or a service policy. Here, the agreement history information D22 includes information related to the presence or absence of agreement of the user in providing, to the medical institution, the vehicle data D1, the user data D2, information related to the content or the like of the abnormal driving detected by the detection device 50 (the abnormality detection data D4).

The service setting information D23 is setting information necessary for enjoying a service registered by the user himself or herself, and is, for example, information such as a language setting and a notification setting in various services. Here, the service setting information D23 includes terminal registration information of the user terminal 60. The terminal registration information of the user terminal 60 is information related to the user terminal 60 registered by the user himself or herself or automatically generated on the integration server 40 side, and is information capable of specifying the user terminal 60 of a notification destination when the detection device 50 issues the medical information notification. The contract package information D24 is information related to a contract automatically generated on the integration server 40 side, and is, for example, information such as a contract status (valid/expiration/cancellation), a contract start date/expiration date/cancellation date, and a contract plan. The favorite information D25 is information related to a preference of the user registered by the user himself or herself, and is, for example, information related to a nearest comprehensive hospital, a home hospital, a medical institution that the user wants to have a consultation, an insurance company that is under contract, and a favorite agency (dealer). In addition, for example, information such as an acquisition date and time of the information is added to the user data D2.

### <Terminal Data>

The terminal data D3 is data related to a transmission history of the medical information notification to the user terminal 60, a transmission history of the abnormality detection data D4 to the medical institution terminal 70, and a history of receiving the medical information data D5 from the medical institution terminal 70. Typically, the terminal data D3 is data collected in the service providing device 30 in accordance with transmission of data to the user terminal 60 and the medical institution terminal 70 and reception of the medical information data D5 from the medical institution terminal 70. The terminal data D3 includes, for example, data related to terminal basic information D30, transmission history information D31, reception history information D32, service providing history information D33, and the like.

The terminal basic information D30 is basic information capable of specifying the user terminal 60 and the medical institution terminal 70, and includes information such as a type of each terminal, an owner of each terminal, and an institution that owns each terminal. The transmission history information D31 is information related to a transmission history when the abnormality detection data D4 is transmitted from the service providing device 30 to the medical institution terminal 70. The reception history information D32 is information related to a reception history when the service providing device 30 receives the medical information data D5 from the medical institution terminal 70. The service providing history information D33 is information related to a history of the service provided by the service providing device 30 to the user (the user terminal 60). The service providing history information D33 in the present embodiment is information related to a history in which the service providing device 30 notifies the user of the medical information having the content based on the medical information data D5. In addition, for example, information such as an acquisition date and time of the information is added to the terminal data D3.

### <Abnormality Detection Data>

The abnormality detection data D4 is data generated when the detection device 50 detects abnormal driving of the user. The abnormality detection data D4 includes abnormality detection information D41. The abnormality detection information D41 is information related to the content of the abnormal driving of the user detected by the detection device 50. For example, the abnormality detection information D41 includes information related to the number of times of sudden braking, sudden steering, overlooking of a temporary stop sign, and the like within a predetermined period (for example, within a period where the vehicle V travels 100 km), a surrounding situation of the vehicle V in a case where the sudden braking, sudden steering, overlooking of a temporary stop sign, and the like occur, and a state of the user captured by an in-vehicle camera during driving. The abnormality detection information D41 may, for example, include information related to an inter-vehicle distance when the inter-vehicle distance to the preceding vehicle is shortened, a surrounding situation of the vehicle V when the inter-vehicle distance to the preceding vehicle is shortened, and the state of the user captured by the in-vehicle camera during driving. The abnormality detection data D4 may further include biological information of the user (driver) when the detection device 50 detects the abnormal driving of the user. Here, the biological information of the user may include, in addition to various kinds of physiological information issued by a living body (user), various kinds of information derived from the physiological information, and is, for example, vital sign information such as a heart rate, a respiratory rate, a pulse rate, a blood pressure, and a body temperature, biological identification information such as a fingerprint, a voiceprint, and an iris, and information such as a blood alcohol concentration and an arousal level. The abnormality detection information D41 may include information such as a date and time when the abnormal driving of the user is detected.

### <Medical Information Data>

The medical information data D5 is data created based on the abnormality detection data D4 and the opinion of the medical worker such as a doctor in the medical institution. The medical information data D5 includes, for example, determination result information D51, treatment program information D52, and consultation recommendation information D53. The determination result information D51 is information related to a determination result related to the cognitive function of the user and obtained by the medical worker such as a doctor of the medical institution. The determination result information D51 includes, for example, information related to the presence or absence, the content, and the like of the indication or the symptom of the disease related to the cognitive function of the user. The treatment program information D52 is information related to therapy determined to be suitable for the user by the medical worker such as a doctor of the medical institution. The treatment program information D52 includes, for example, information related to therapy selected by the medical worker from the medication therapy and the occupational therapy such as exercise therapy, reminiscence therapy, music therapy, and cognitive stimulation therapy. Furthermore, the treatment program information D52 may include, for example, information such as a specific content of the therapy selected by the medical worker, a location of a medical institution where the user can consult the therapy, an assumed treatment schedule, and a type of medicine considered to be suitable for the user. The consultation recommendation information D53 is information related to the consultation recommendation for the disease related to the cognitive function. The consultation recommendation information D53 includes, for example, information related to the consultation recommendation to the medical institution or the consultation recommendation of the cognitive function inspection. The consultation recommendation information D53 is, for example, information related to the consultation recommendation to the specific medical institution determined to be suitable for the user by the medical worker.

### <ID Data>

The ID data D6 is an identifier that can manage the vehicle data D1, the user data D2, the terminal data D3, the abnormality detection data D4, and the medical information data D5 described above in association with each other. The ID data D6 includes, for example, data related to a user ID D60 and a vehicle ID D61. The user ID D60 is a unique identifier assigned to each user to identify the user. The user data D2, the terminal data D3, the abnormality detection data D4, and the medical information data D5 described above are managed in association with the user ID D60. On the other hand, a vehicle ID D61 is a unique identifier assigned to each vehicle V in order to identify the vehicle V Here, in order to facilitate identification and management of the vehicle V, the vehicle ID D61 is set separately from the vehicle identification information D10 described above. The vehicle data D1 described above is managed in association with the vehicle ID D61.

The user ID D60 and the vehicle ID D61 are associated with each other in correspondence with the vehicle V used by the user. For example, when one user owns a plurality of vehicles V, a relation between the user ID D60 and the vehicle ID D61 is that a plurality of vehicle IDs D61 are associated with one user ID D60. As a result, the vehicle data D1, the user data D2, the terminal data D3, the abnormality detection data D4, and the medical information data D5 can be associated with each other for each vehicle corresponding to each vehicle ID D61 based on the user ID D60 or the like. That is, the vehicle data D1 related to the vehicle V used by a specific user, and the user data D2, the terminal data D3, the abnormality detection data D4, and the medical information data D5 related to the specific user can be managed as a set of data sets associated with each other via the user ID D60 and the vehicle IDs D61. As described above, when the plurality of vehicle IDs D61 are associated with one user ID D60, a plurality of pieces of vehicle data D1 are respectively associated with the user data D2 of the corresponding user, the terminal data D3, the abnormality detection data D4, and the medical information data D5 via the user ID D60 and the vehicle IDs D61. On the other hand, when one vehicle V is used by a plurality of users, a relation between the user ID D60 and the vehicle ID D61 is that a plurality of user IDs D60 are associated with one vehicle ID D61.

An overview of the main data treated by the abnormality detection system 1 has been described above. Although the abnormality detection system 1 also treats data other than the data described above, the data will be appropriately described later.

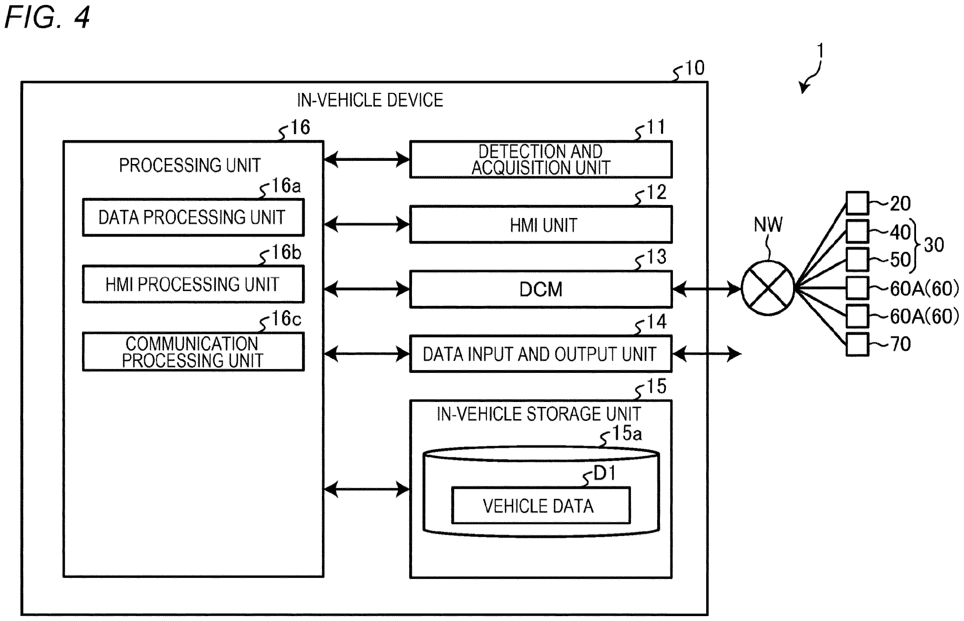

Next, configurations of the abnormality detection system 1 will be described with reference to FIGS. 4 to 12. In the following description, various kinds of data may be appropriately referred to in FIG. 3.

### <Basic Configuration of In-vehicle Device>

The in-vehicle device 10 shown in FIG. 4 is a device that is mounted on the vehicle V and that collects and stores the vehicle data D1 related to the vehicle V in association with the vehicle identification information D10 of the vehicle V The in-vehicle device 10 is capable of communicating with an external device of the vehicle V. For example, the in-vehicle device 10 may include a function of a so-called drive recorder or the like that is mounted on the vehicle V and records various kinds of information, and may be configured to be installed in the vehicle V later.

Specifically, as shown in FIG. 4, the in-vehicle device 10 includes a detection and acquisition unit 11, a human machine interface (HMI) unit 12, a DCM 13, a data input and output unit 14, an in-vehicle storage unit 15, and a processing unit 16. The detection and acquisition unit 11, the HMI unit 12, the DCM 13, the data input and output unit 14, the in-vehicle storage unit 15, and the processing unit 16 are communicably connected to each other.

The detection and acquisition unit 11 is a device that detects and acquires various kinds of information included in the vehicle data D1 related to the vehicle V The detection and acquisition unit 11 detects and acquires the vehicle identification information D10, the DCM information D11, the vehicle basic information D12, the traveling information D13, and the like as the vehicle data D1. The detection and acquisition unit 11 includes, for example, various sensors provided in units of the vehicle V, a detector, a GPS receiver, a microphone, and an in-vehicle camera. The detection and acquisition unit 11 may include, for example, a device that acquires various kinds of information from an electronic control unit (ECU) that integrally controls the entire vehicle V. When the biological information or the like of the user (driver) of the vehicle V is included in the vehicle data D1, the detection and acquisition unit 11 may include a device that acquires various kinds of information in cooperation with a monitoring device that monitors the user or a wearable terminal worn by the user.

The HMI unit 12 is a device that performs various inputs and outputs in the in-vehicle device 10. The HMI unit 12 may include, for example, a keyboard, a switch, an input button, a mouse pointer, a trackball, a joystick, a touch pad, a microphone, and a voice input device, as an operation input device that performs various inputs to the in-vehicle device 10. The HMI unit 12 may include, for example, a speaker and a display, as an information output device that performs various outputs from the in-vehicle device 10.

The DCM 13 is a communication module capable of communicating with an external device of the vehicle V The DCM 13 is communicably connected to the network NW by wireless communication, and communicates with the external device of the vehicle V (typically, the data processing device 20) via the network NW.

The data input and output unit 14 is a data input and output device that inputs and outputs data (information) to and from the in-vehicle device 10. The data input and output unit 14 is implemented by a recording medium interface or the like, and reads and writes various kinds of data from and to a recording medium such as a flexible disk (FD), a solid state drive (SSD), a hard disk drive (HDD), a magneto-optical disk, a USB memory, an SD card memory, a flash memory, a CD-ROM, or a DVD.

The in-vehicle storage unit 15 is a storage circuit that stores various kinds of data. The in-vehicle storage unit 15 may be, for example, a storage device having a relatively large capacity such as an HDD, an SSD, or an optical disk, or a semiconductor memory capable of rewriting data such as a RAM, a flash memory, or a non volatile static random access memory (NVSRAM). The in-vehicle storage unit 15 stores, for example, programs that enable units of the in-vehicle device 10 to implement various functions. The in-vehicle storage unit 15 stores various kinds of data necessary for various kinds of processes in the processing unit 16, and the processing unit 16 or the like reads the various kinds of data as necessary.

The in-vehicle storage unit 15 according to the present embodiment includes a vehicle data storage unit 15a in terms of functional concept.

The vehicle data storage unit 15a is a storage area for storing the vehicle data D1 in the in-vehicle storage unit 15. The vehicle data storage unit 15a stores, as the vehicle data D1, data related to the vehicle identification information D10, the DCM information D11, the vehicle basic information D12, the traveling information D13, and the like detected and acquired by the detection and acquisition unit 11.

The processing unit 16 is a processing circuit that integrally controls the in-vehicle device 10 and implements various processing functions of the in-vehicle device 10. The processing unit 16 is implemented by, for example, a processor, a RAM, and a ROM. The processor means a circuit such as a central processing unit (CPU), a micro processing unit (MPU), a field programmable gate array (FPGA), or an application specific integrated circuit (ASIC). The processing unit 16 implements each processing function, for example, by executing a program read from the in-vehicle storage unit 15. For example, the processing unit 16 is capable of executing a process of detecting and acquiring the vehicle data D1 by the detection and acquisition unit 11 and storing the vehicle data D1 in the vehicle data storage unit 15a, a process of operating the HMI unit 12, a process of performing data communication via the DCM 13, and the like.

The processing unit 16 according to the present embodiment includes a data processing unit 16a, an HMI processing unit 16b, and a communication processing unit 16c in terms of functional concept in order to implement the various processing functions described above. The processing unit 16 implements processing functions of the data processing unit 16a, the HMI processing unit 16b, and the communication processing unit 16c, for example, by reading and executing the programs (not shown) stored in the in-vehicle storage unit 15.

The data processing unit 16a is a unit having a function capable of executing a process related to various kinds of data in the in-vehicle device 10. The data processing unit 16a is capable of executing a process of detecting and acquiring information constituting the vehicle data D1 by the detection and acquisition unit 11. The data processing unit 16a is capable of executing a process of storing, in the vehicle data storage unit 15a, information detected or acquired by the detection and acquisition unit 11 as the vehicle data D1. The data processing unit 16a is capable of executing a process of reading the vehicle data D1 from the vehicle data storage unit 15a in response to a request. The data processing unit 16a is capable of executing a process of inputting or outputting data to or from the in-vehicle device 10 via the data input and output unit 14. The data processing unit 16a is also capable of executing a process of storing data input via the data input and output unit 14 in the in-vehicle storage unit 15 and a process of reading, from the in-vehicle storage unit 15, data output via the data input and output unit 14. The data processing unit 16a is also capable of executing a process of storing data registered in accordance with an operation on an operation input device constituting the HMI unit 12 in the in-vehicle storage unit 15 and a process of reading the data from the in-vehicle storage unit 15.

For example, the data processing unit 16a may control the detection and acquisition unit 11 to detect and acquire information constituting the vehicle data D1 at a predetermined sampling cycle, or may detect and acquire the information at an appropriate timing. The data processing unit 16a may acquire a part of the vehicle data D1, such as the vehicle identification information D10, the DCM information D11, and the vehicle basic information D12, registered in accordance with an operation to the operation input device constituting the HMI unit 12. When storing the vehicle data D1 in the vehicle data storage unit 15a, the data processing unit 16a stores the vehicle identification information D10 of the vehicle V on which the in-vehicle device 10 is mounted and the vehicle data D1 in association with each other in the vehicle data storage unit 15a. In this case, for example, the data processing unit 16a can store, in the vehicle data storage unit 15a, the vehicle data D1 along a time series together with information on a date and time when the vehicle data D1 is collected or the like.

The HMI processing unit 16b is a unit having a function capable of executing a process of controlling the HMI unit 12 and operating the HMI unit 12. The HMI processing unit 16b is capable of executing a process of controlling the operation input device constituting the HMI unit 12 and inputting an operation via the operation input device. The HMI processing unit 16b is capable of executing a process of controlling the information output device constituting the HMI unit 12 and outputting information via the information output device.

The communication processing unit 16c is a unit having a function capable of executing a process of controlling the DCM 13 and performing data communication. The communication processing unit 16c is capable of executing a process of transmitting and receiving data to and from other devices such as the data processing device 20 via the DCM 13. More specifically, the communication processing unit 16c is capable of executing a process of transmitting the vehicle data D1 to the data processing device 20 via the DCM 13 in a state where the vehicle identification information D10 and the corresponding vehicle data D1 are associated with each other. The communication processing unit 16c is capable of executing a process of receiving various commands, requests, and various kinds of information from the data processing device 20 via the DCM 13. The communication processing unit 16c is capable of executing a process of transmitting various commands, requests, and various kinds of information to the data processing device 20 via the DCM 13.

For example, the communication processing unit 16c can transmit the vehicle data D1 stored in the vehicle data storage unit 15a to the data processing device 20 at an appropriate timing. In this case, for example, the communication processing unit 16c may sequentially transmit the vehicle data D1 to the data processing device 20 in real time in accordance with a sampling cycle of the detection and acquisition unit 11, or may collectively transmit the vehicle data D1 of a plurality of sampling cycles to the data processing device 20 at a cycle different from the sampling cycle of the detection and acquisition unit 11. For example, the communication processing unit 16c may transmit the vehicle data D1 to the data processing device 20 at any timing in accordance with an operation on the operation input device constituting the HMI unit 12, or may transmit the vehicle data D1 to the data processing device 20 at a timing when a request such as a transmission command from an external device (the data processing device 20 or the like) is received. In this case, for example, the in-vehicle device 10 can be expected to have an effect of reducing congestion due to an increase in a communication traffic amount. For example, the communication processing unit 16c can change a transmission cycle of the vehicle data D1 according to the state of the vehicle V In this case, for example, when the vehicle V is stopped, a significant change in the vehicle speed, the position information, or the like is not expected, and thus the communication processing unit 16c may transmit the vehicle data D1 at a transmission cycle that is relatively longer than a transmission cycle when the vehicle V is traveling. In this case, for example, the in-vehicle device 10 can transmit data at an appropriate timing in accordance with the state of the vehicle V

### <Basic Configuration of Data Processing Device>

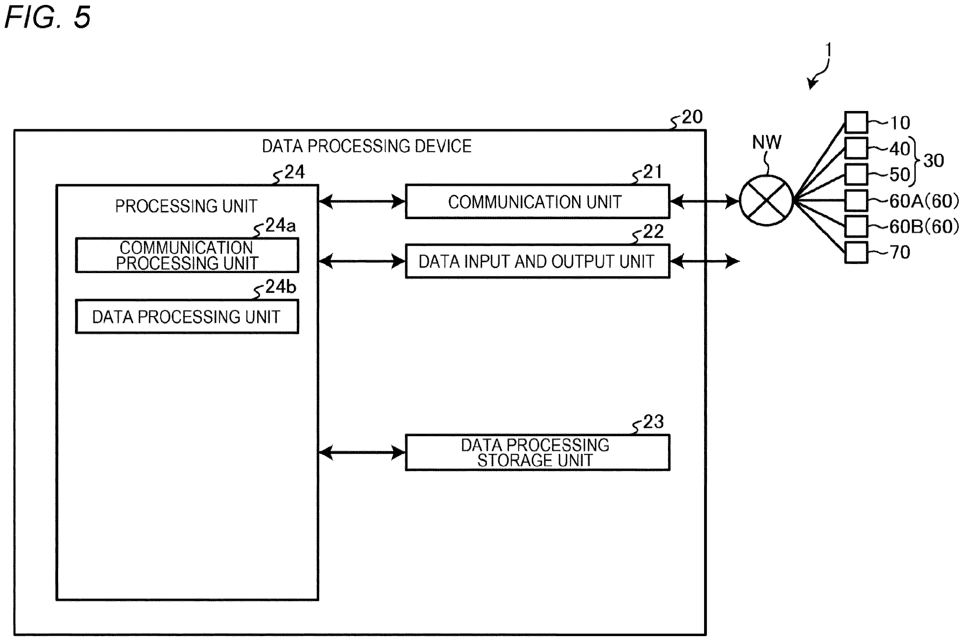

The data processing device 20 shown in FIG. 5 is a network relay device that relays data communication between the in-vehicle device 10 and the service providing device 30 (for example, the integration server 40). In the present embodiment, the data processing device 20 relays data to be transmitted from the in-vehicle device 10 to the service providing device 30. The service providing device 30 stores the data received from the data processing device 20 in the integration server 40. The data processing device 20 may be implemented by, for example, various computer devices. The data processing device 20 can also be implemented by, for example, installing programs for realizing various processes in a known computer device.

Specifically, as shown in FIG. 5, the data processing device 20 includes a communication unit 21, a data input and output unit 22, a data processing storage unit 23, and a processing unit 24. The communication unit 21, the data input and output unit 22, the data processing storage unit 23, and the processing unit 24 are communicably connected to each other.

The communication unit 21 is a communication module capable of communicating with devices other than the data processing device 20. The communication unit 21 is communicably connected to the network NW regardless of wireless communication or wired communication, and communicates with other devices (for example, the in-vehicle device 10 and the service providing device 30) via the network NW.

The data input and output unit 22 is a data input and output device that inputs and outputs data (information) to and from the data processing device 20. The data input and output unit 22 has substantially the same configuration as the data input and output unit 14 described above, and is implemented by a recording medium interface or the like.

The data processing storage unit 23 is a storage circuit that stores various kinds of data. The data processing storage unit 23 has substantially the same configuration as the in-vehicle storage unit 15 described above, and is implemented by, for example, an HDD, an SSD, an optical disk, and a semiconductor memory. The data processing storage unit 23 stores, for example, programs that enable units of the data processing device 20 to implement various functions. The data processing storage unit 23 stores various kinds of data necessary for various kinds of processes in the processing unit 24, and the processing unit 24 or the like reads the various kinds of data as necessary.

The processing unit 24 is a processing circuit that integrally controls the data processing device 20 and implements various processing functions of the data processing device 20. The processing unit 24 has substantially the same configuration as the processing unit 16 described above, and is implemented by, for example, a processor, a RAM, and a ROM. The processing unit 24 implements processing functions, for example, by reading and executing the programs stored in the data processing storage unit 23. For example, the processing unit 24 is capable of executing a process of performing data communication via the communication unit 21, a process of relaying various kinds of data, a process of transmitting data received from the in-vehicle device 10 to the integration server 40 and storing the data, and the like.

The processing unit 24 according to the present embodiment includes a communication processing unit 24a and a data processing unit 24b in terms of functional concept in order to implement the various processing functions described above. The processing unit 24 implements processing functions of the data processing unit 24b and the communication processing unit 24a by reading and executing a program stored in the data processing storage unit 23, for example.

The communication processing unit 24a is a unit having a function capable of executing a process of controlling the communication unit 21 and performing data communication. The communication processing unit 24a is capable of executing a process of transmitting and receiving data to and from other devices such as the in-vehicle device 10 and the service providing device 30 via the communication unit 21. More specifically, for example, the communication processing unit 24a is capable of executing a process of receiving the vehicle data D1 from the in-vehicle device 10 via the communication unit 21. The communication processing unit 24a is capable of executing a process of transmitting the vehicle data D1 to the service providing device 30 (for example, the integration server 40) via the communication unit 21. The communication processing unit 24a is capable of executing a process of receiving various commands and requests from the in-vehicle device 10, the service providing device 30, and the like via the communication unit 21. The communication processing unit 24a is capable of executing a process of transmitting various commands, requests, and various kinds of information to the in-vehicle device 10 and the service providing device 30 (for example, the integration server 40) via the communication unit 21.

For example, when relaying data between the devices, the communication processing unit 24a executes processes such as a process of blocking unauthorized communication, a process of ensuring communication with a high security level such as encrypted communication, a routing process of distributing a transmission destination of data, and a protocol conversion process of converting a communication protocol between different networks.

The data processing unit 24b is a unit having a function capable of executing a process related to various kinds of data in the data processing device 20. The data processing unit 24b is capable of executing a process of storing data received via the communication unit 21 in the data processing storage unit 23. The data processing unit 24b is capable of executing a process of reading data transmitted via the communication unit 21 from the data processing storage unit 23. The data processing unit 24b is capable of executing a process of inputting and outputting data to and from the data processing device 20 via the data input and output unit 22. The data processing unit 24b is also capable of executing a process of storing data input via the data input and output unit 22 in the data processing storage unit 23 and a process of reading data output via the data input and output unit 22 from the data processing storage unit 23.

The communication processing unit 24a transmits the vehicle data D1 read from the data processing storage unit 23 by the data processing unit 24b to the integration server 40 via the communication unit 21.

### <Basic Configuration of Service Providing Device>

The service providing device 30 is capable of comprehensively managing various kinds of data treated by the abnormality detection system 1, detecting the abnormal driving of the user based on the vehicle data D1, storing the abnormality detection data D4 including the information related to the content of the detected abnormal driving, and transmitting the abnormality detection data D4 and the user data D2 in association with each other to the medical institution terminal 70 provided in the medical institution if the abnormal driving of the user in the vehicle V is detected. As shown in FIG. 2, the service providing device 30 includes the integration server 40 and the detection device 50. Typically, the service providing device 30 constitutes a base station device provided in a base station 100 that relays communication between the vehicle V and the user terminal 60 or the medical institution terminal 70, and is managed by, for example, a business operator that develops various services related to medical care or a business operator that develops the vehicle V The detection device 50 does not need to be disposed in the base station 100, and may be managed in a place different from the base station 100. For example, the detection device 50 may be configured to access the base station device from a remote location different from a location of the base station 100. That is, the base station device may be implemented by the integration server 40 at least. A device in which functions of the integration server 40 and the detection device 50 are integrated into one device may be utilized as the service providing device 30.

In the service providing device 30 according to the present embodiment, the detection device 50 is configured to communicate with the integration server 40 and the medical institution terminal 70, and the integration server 40 is configured to communicate with the detection device 50 and the user terminal 60. In this way, the service providing device 30 according to the present embodiment is configured such that the medical institution terminal 70 cannot directly access the integration server 40. In the service providing device 30, the abnormality detection data D4 is transmitted from the detection device 50 to the medical institution terminal 70. In the service providing device 30, the detection device 50 receives the medical information data D5 from the medical institution terminal 70. The detection device 50 notifies the user terminal 60 of the medical information including the content based on the medical information data D5 received from the medical institution terminal 70. In this case, the medical information is transmitted from the detection device 50 to the user terminal 60 via the integration server 40.

The integration server 40 and the detection device 50 are implemented by, for example, various computer devices. The integration server 40 and the detection device 50 may be implemented by, for example, installing programs for realizing various processes in a known computer device. Here, the integration server 40 is physically implemented as one server.

Hereinafter, configurations of the integration server 40 and the detection device 50 will be described.

### <Basic Configuration of Integration Server>

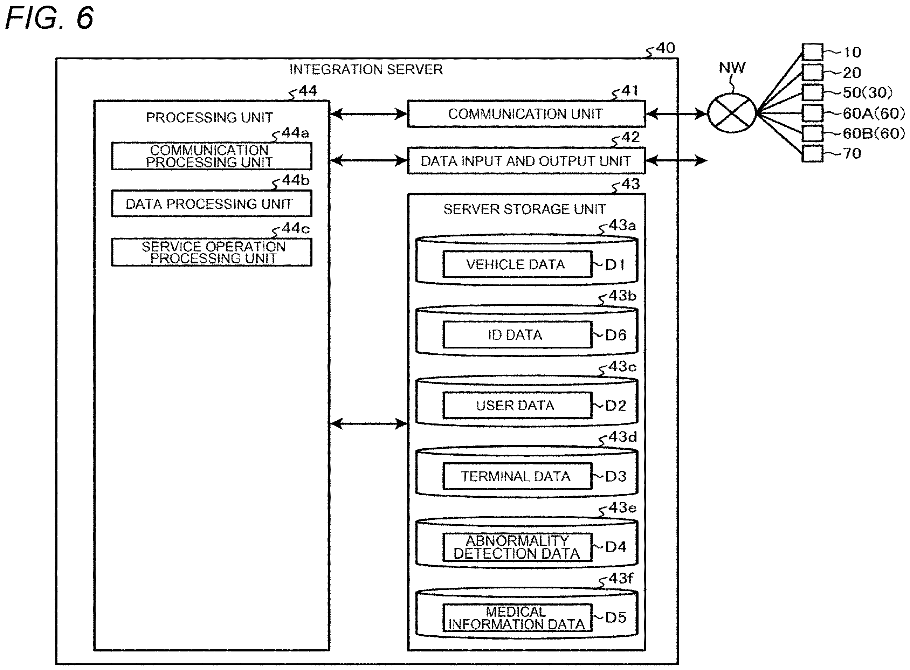

The integration server 40 shown in FIG. 6 is a server that manages data mainly used for a service or the like provided to the user of the vehicle V The integration server 40 may also be referred to as a connected server that develops a connected service. In the abnormality detection system 1 according to the present embodiment, a service is performed in which the data stored in the integration server 40 is used to detect the abnormal driving of the user who drives the vehicle V, and the medical information created based on the information related to the detected abnormal driving of the user and the opinion of the medical worker of the medical institution is notified to the user terminal 60. In addition to this service, other services may be developed using data stored in the integration server 40. Examples of the other services deployed by using the data stored in the integration server 40 include, but are not limited to, contract management, parking position search, vehicle remote control, emergency notification support, trouble support, security notification, and driving history evaluation. Each service may include a service linked to an application function of the user terminal 60.

Specifically, as shown in FIG. 6, the integration server 40 includes a communication unit 41, a data input and output unit 42, a server storage unit 43, and a processing unit 44. The communication unit 41, the data input and output unit 42, the server storage unit 43, and the processing unit 44 are communicably connected to each other.

The communication unit 41 is a communication module capable of communicating with devices other than the integration server 40. The communication unit 41 has substantially the same configuration as the communication unit 21 described above, is communicably connected to the network NW regardless of wireless communication or wired communication, and communicates with other devices (for example, the data processing device 20, the detection device 50, and the user terminal 60) via the network NW.

The data input and output unit 42 is a data input and output device that inputs and outputs data (information) to and from the integration server 40. The data input and output unit 42 has substantially the same configuration as the data input and output unit 14 described above, and is implemented by a recording medium interface or the like.

The server storage unit 43 is a storage circuit that stores various kinds of data. The server storage unit 43 has substantially the same configuration as the in-vehicle storage unit 15 described above, and is implemented by, for example, an HDD, an SSD, an optical disk, and a semiconductor memory. The server storage unit 43 stores, for example, programs that enable units of the integration server 40 to implement various functions. The server storage unit 43 stores various kinds of data necessary for various kinds of processes in the processing unit 44, and the processing unit 44 or the like reads the various kinds of data as necessary.

The server storage unit 43 according to the present embodiment includes a vehicle data storage unit 43a, an ID data storage unit 43b, a user data storage unit 43c, a terminal data storage unit 43d, an abnormality detection data storage unit 43e, and a medical information data storage unit 43f in terms of functional concept.

The vehicle data storage unit 43a is a storage circuit that stores various kinds of data. The vehicle data storage unit 43a has substantially the same configuration as the in-vehicle storage unit 15 described above, and is implemented by, for example, an HDD, an SSD, an optical disk, and a semiconductor memory. The vehicle data storage unit 43a is a storage area for storing the vehicle data D1 in the server storage unit 43. The vehicle data storage unit 43a stores the vehicle data D 1 transmitted from the in-vehicle device 10. The vehicle data storage unit 43a stores, for example, the vehicle data D1 including data related to the vehicle identification information D10, the DCM information D11, the vehicle basic information D12, the traveling information D13, and the like.

The ID data storage unit 43b is a storage area for storing the ID data D6 in the server storage unit 43. The ID data storage unit 43b stores, for example, the ID data D6 including data related to the user ID D60, the vehicle ID D61, and the like registered via the user terminal 60 or automatically generated by the processing unit 44 of the integration server 40.

The user data storage unit 43c is a storage area for storing the user data D2 in the server storage unit 43. The user data storage unit 43c stores, for example, the user data D2 including data related to the user basic information D20, the user attribute information D21, the agreement history information D22, the service setting information D23, the contract package information D24, the favorite information D25, and the like registered via the in-vehicle device 10, the user terminal 60, or the like, or automatically generated by the processing unit 44 of the integration server 40.

The terminal data storage unit 43d is a storage area for storing the terminal data D3 in the server storage unit 43. The terminal data storage unit 43d stores, for example, the terminal data D3 including data related to the terminal basic information D30, the transmission history information D31, the reception history information D32, the service providing history information D33, and the like of the user terminal 60.

The abnormality detection data storage unit 43e is a storage area for storing the abnormality detection data D4 in the server storage unit 43. That is, the abnormality detection data storage unit 43e is a storage area for storing the abnormality detection data D4 transmitted from the detection device 50 to the integration server 40. The abnormality detection data storage unit 43e stores the abnormality detection data D4 including data related to the abnormality detection information D41.

The medical information data storage unit 43f is a storage area for storing the medical information data D5 in the server storage unit 43. That is, the medical information data storage unit 43f is a storage area for storing the medical information data D5 transmitted from the medical institution terminal 70 to the integration server 40 via the detection device 50. The medical information data storage unit 43f stores, for example, the medical information data D5 including data related to the determination result information D51, the treatment program information D52, the consultation recommendation information D53, and the like.

The processing unit 44 is a processing circuit that integrally controls the integration server 40 and implements various processing functions of the integration server 40. The processing unit 44 has substantially the same configuration as the processing unit 16 described above, and is implemented by, for example, a processor, a RAM, and a ROM. The processing unit 44 implements processing functions, for example, by reading and executing the program stored in the server storage unit 43. For example, the processing unit 44 is capable of executing a process of performing data communication via the communication unit 41, a process of specifying the user data D2 corresponding to the abnormality detection data D4, a process of specifying the user data D2 corresponding to the medical information data D5, a process of calculating various kinds of data, a process of providing various services to the user, and the like.

The processing unit 44 according to the present embodiment includes a communication processing unit 44a, a data processing unit 44b, and a service operation processing unit 44c in terms of functional concept in order to implement the various processing functions described above. The processing unit 44 implements processing functions of the communication processing unit 44a, the data processing unit 44b, and the service operation processing unit 44c, for example, by reading and executing the program stored in the server storage unit 43.

The communication processing unit 44a is a unit having a function capable of executing a process of controlling the communication unit 41 and performing data communication. The communication processing unit 44a is capable of executing a process of transmitting and receiving data to and from other devices such as the data processing device 20 and the user terminal 60 via the communication unit 41.

For example, the communication processing unit 44a is capable of executing a process of receiving the ID data D6 from the user terminal 60 or the like via the communication unit 41. The communication processing unit 44a is capable of executing a process of receiving the vehicle data D1 transmitted from the data processing device 20 to the integration server 40 via the communication unit 41. The communication processing unit 44a is capable of executing a process of receiving the user data D2 from the in-vehicle device 10 and the user terminal 60 via the communication unit 41. The communication processing unit 44a is capable of executing a process of receiving the abnormality detection data D4 and the medical information data D5 from the detection device 50 via the communication unit 41. The communication processing unit 44a is capable of executing a process of transmitting the medical information notification to the user terminal 60 via the communication unit 41 in response to a request received from the detection device 50. The communication processing unit 44a is capable of executing a process of receiving various commands and requests from the data processing device 20, the detection device 50, the user terminal 60, and the like via the communication unit 41.

The data processing unit 44b is a unit having a function capable of executing a process related to various kinds of data in the integration server 40. The data processing unit 44b is capable of executing a process of storing data received via the communication unit 41 in the server storage unit 43. The data processing unit 44b is capable of executing a process of reading data transmitted via the communication unit 41 from the server storage unit 43. The data processing unit 44b is also capable of executing a process of storing data input via the data input and output unit 42 in the server storage unit 43 and a process of reading data output via the data input and output unit 42 from the server storage unit 43.

More specifically, for example, the data processing unit 44b is capable of executing a process of storing the vehicle data D1 transmitted from the in-vehicle device 10 via the data processing device 20 in the vehicle data storage unit 43a. The data processing unit 44b is capable of executing a process of reading the vehicle data D1 from the vehicle data storage unit 43a in response to a request.

The data processing unit 44b is capable of executing a process of storing, in the ID data storage unit 43b, data related to the user ID D60, the vehicle ID D61, or the like registered via the user terminal 60 or the like as the ID data D6. The data processing unit 44b may execute a process of automatically generating data related to the user ID D60, the vehicle ID D61, and the like based on information registered via the user terminal 60 or the like, and storing the generated data as the ID data D6 in the ID data storage unit 43b. The data processing unit 44b is capable of executing a process of reading the ID data D6 from the ID data storage unit 43b in response to the request.

The data processing unit 44b is capable of executing a process of storing, in the user data storage unit 43c, data related to the user basic information D20, the service setting information D23, the favorite information D25, and the like registered via the user terminal 60 or the like as the user data D2. The data processing unit 44b is capable of executing a process of automatically generating data related to a part of the user data D2 stored in the integration server 40, that is, the user attribute information D21, the agreement history information D22, the contract package information D24, and the like based on information registered via the user terminal 60 or the like, and storing the generated data as the user data D2 in the user data storage unit 43c. For example, the data processing unit 44b may store data registered via the in-vehicle device 10 as a part of the user data D2 in the user data storage unit 43c and form the user data D2. The data processing unit 44b is capable of executing a process of reading the user data D2 from the user data storage unit 43c in response to a request.

The data processing unit 44b is capable of executing a process of storing, in the terminal data storage unit 43d, data related to the terminal basic information D30 transmitted from the user terminal 60, the medical institution terminal 70, or the like as the terminal data D3. The data processing unit 44b is capable of executing a process of acquiring data related to the transmission history information D31, the reception history information D32, the service providing history information D33, and the like from the detection device 50 or the like, generating the data as the terminal data D3, and storing the generated data in the terminal data storage unit 43d. The data processing unit 44b is capable of executing a process of reading the terminal data D3 from the terminal data storage unit 43d in response to a request.

As described above, when respectively storing the vehicle data D1, the user data D2, the terminal data D3, the abnormality detection data D4, and the medical information data D5 in the vehicle data storage unit 43a, the user data storage unit 43c, the terminal data storage unit 43d, the abnormality detection data storage unit 43e, and the medical information data storage unit 43f, the data processing unit 44b is capable of executing a process of associating the vehicle data D1, the user data D2, the terminal data D3, and the abnormality detection data D4, and the medical information data D5 with each other. For example, the data processing unit 44b associates, via the vehicle identification information D10 included in the vehicle data D1 and the user ID D60 and the vehicle ID D61 constituting the ID data D6, the vehicle data D1, the user data D2, the terminal data D3, the abnormality detection data D4, and the medical information data D5 stored in the vehicle data storage unit 43a, the user data storage unit 43c, the terminal data storage unit 43d, the abnormality detection data storage unit 43e, and the medical information data storage unit 43f with each other to form a data set.

That is, the server storage unit 43 stores the vehicle data D1 stored in the vehicle data storage unit 43a, the user data D2 stored in the user data storage unit 43c, the terminal data D3 stored in the terminal data storage unit 43d, the abnormality detection data D4 stored in the abnormality detection data storage unit 43e, and the medical information data D5 stored in the medical information data storage unit 43f by the data processing unit 44b associating the above data with each other based on the vehicle identification information D10 and the ID data D6, and stores the above data in a database as a data set.

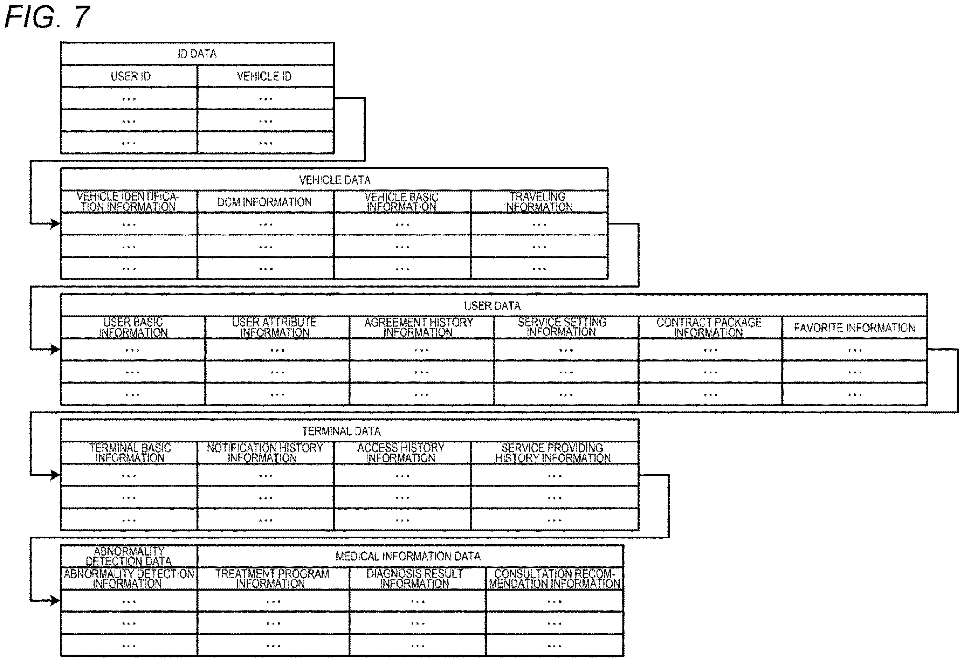

FIG. 7 is an example of a database stored in the integration server (storage unit) 40. For example, as shown in FIG. 7, the integration server 40 stores the vehicle data D1, the user data D2, the terminal data D3, the abnormality detection data D4, the medical information data D5, and the ID data D6 as a database in a table format in which the above data are associated with each other.

The service operation processing unit 44c is a unit having a function capable of executing a process related to provision of a service based on commands and requests received from the data processing device 20, the detection device 50, the user terminal 60, and the like, and based on data stored in the server storage unit 43. For example, the service operation processing unit 44c transmits the vehicle data D1 to the detection device 50 in response to a command and a request received from the detection device 50. For example, the service operation processing unit 44c specifies a user (a user terminal) as a target of the medical information notification from the user data D2 corresponding to the medical information data D5 received from the detection device 50 in response to a command or a request received from the detection device 50, and transmits the medical information notification to the user. For example, the service operation processing unit 44c is capable of executing some or all of the services such as the contract management, the parking position search, the vehicle remote control, the emergency notification support, the trouble support, the security notification, and the driving history evaluation described above. The service operation processing unit 44c may execute the services in cooperation with the user terminal 60 or other external terminals. When performing an operation related to provision of a service, the service operation processing unit 44c appropriately reads data necessary for executing the requested service from the vehicle data D1, the user data D2, the terminal data D3, the abnormality detection data D4, and the medical information data D5 stored in the server storage unit 43, and operates to provide the service to the user based on the data.

### <Basic Configuration of Detection Device>

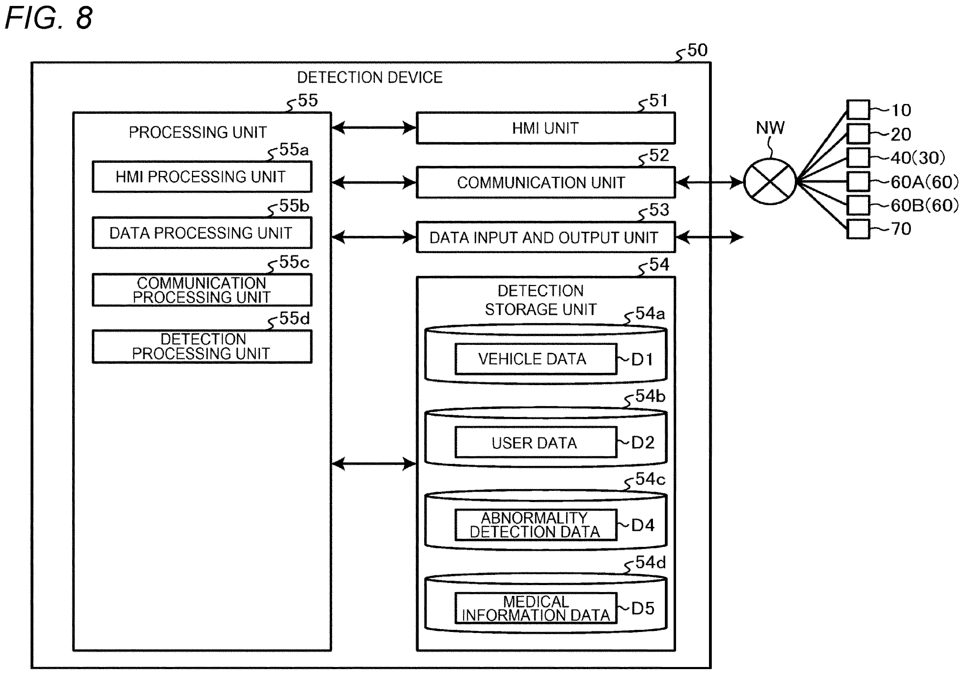

The detection device 50 shown in FIG. 8 acquires the vehicle data D1 from the integration server 40, detects the abnormal driving of the user in the vehicle V based on the acquired vehicle data D1, and transmits the abnormality detection data D4 including the information related to the content or the like of the detected abnormal driving to the medical institution terminal 70. The detection device 50 transmits the abnormality detection data D4 to the integration server 40 and stores the abnormality detection data D4. The detection device 50 receives, from the medical institution terminal 70, the medical information data D5 created based on the abnormality detection data D4 and the opinion of the medical worker of the medical institution. The detection device 50 notifies the user (the user terminal 60) of the medical information including the content based on the medical information data D5 via the integration server 40. The detection device 50 according to the present embodiment is a device that is managed and used by a business operator that mainly provides various services related to medical care or the business operator that develops the vehicle V The detection device 50 is configured to execute various analyses such as quality investigation, accident analysis, failure analysis, feedback to development, mobility planning, and utilization for new business based on the data stored in the integration server 40.

Specifically, the detection device 50 includes an HMI unit 51, a communication unit 52, a data input and output unit 53, a detection storage unit 54, and a processing unit 55. The HMI unit 51, the communication unit 52, the data input and output unit 53, the detection storage unit 54, and the processing unit 55 are communicably connected to each other.

The HMI unit 51 is a device that performs various inputs and outputs in the detection device 50. Similarly to the HMI unit 12 described above, the HMI unit 51 includes an operation input device that performs various inputs to the detection device 50 and an information output device that performs various outputs from the detection device 50.

The communication unit 52 is a communication module capable of communicating with devices other than the detection device 50. The communication unit 52 has substantially the same configuration as the communication unit 21 described above, is communicably connected to the network NW regardless of wireless communication or wired communication, and communicates with other devices (for example, the data processing device 20, the integration server 40, the user terminal 60, and the medical institution terminal 70) via the network NW.

The data input and output unit 53 is a data input and output device that inputs and outputs data (information) to and from the detection device 50. The data input and output unit 53 has substantially the same configuration as the data input and output unit 14 described above, and is implemented by a recording medium interface or the like.

The detection storage unit 54 is a storage circuit that stores various kinds of data. The detection storage unit 54 has substantially the same configuration as the in-vehicle storage unit 15 described above, and is implemented by, for example, an HDD, an SSD, an optical disk, and a semiconductor memory. The detection storage unit 54 stores, for example, programs that enable units of the detection device 50 to implement various functions. The detection storage unit 54 stores various kinds of data necessary for various kinds of processes in the processing unit 55, and the processing unit 55 or the like reads the various kinds of data as necessary.

The processing unit 55 is a processing circuit that integrally controls the detection device 50 and implements various processing functions of the detection device 50. The processing unit 55 has substantially the same configuration as the processing unit 16 described above, and is implemented by, for example, a processor, a RAM, and a ROM. The processing unit 55 implements processing functions, for example, by reading and executing the program stored in the detection storage unit 54. For example, the processing unit 55 is capable of executing a process of operating the HMI unit 51, a process of performing data communication via the communication unit 52, and the like.

The processing unit 55 according to the present embodiment includes an HMI processing unit 55a, a data processing unit 55b, and a communication processing unit 55c in order to implement the various processing functions of the detection device 50. The processing unit 55 implements processing functions of the HMI processing unit 55a, the data processing unit 55b, and the communication processing unit 55c by reading and executing the program stored in the detection storage unit 54, for example.

Similarly to the HMI processing unit 16b described above, the HMI processing unit 55a is a unit having a function capable of executing a process of controlling the HMI unit 51 and operating the HMI unit 51. The HMI processing unit 55a is capable of executing a process of controlling an operation input device constituting the HMI unit 51 and inputting an operation via the operation input device. The HMI processing unit 55a is capable of executing a process of controlling an information output device constituting the HMI unit 51 and outputting information via the information output device.

Similarly to the data processing unit 44b described above, the data processing unit 55b is a unit having a function capable of executing a process related to various kinds of data in the detection device 50. The data processing unit 55b is capable of executing a process of storing data received via the communication unit 52 in the detection storage unit 54. The data processing unit 55b is capable of executing a process of reading data transmitted via the communication unit 52 from the detection storage unit 54. The data processing unit 55b is also capable of executing a process of storing data input via the data input and output unit 53 in the detection storage unit 54 and a process of reading data output via the data input and output unit 53 from the detection storage unit 54. The data processing unit 55b is also capable of executing a process of storing registered data in the detection storage unit 54 in accordance with an operation on the operation input device constituting the HMI unit 51, and a process of reading the data from the detection storage unit 54.

Similarly to the communication processing unit 44a described above, the communication processing unit 55c is a unit having a function capable of executing a process of controlling the communication unit 52 and performing data communication. The communication processing unit 55c is capable of executing a process of transmitting and receiving data to and from other devices such as the data processing device 20, the integration server 40, the user terminal 60, and the medical institution terminal 70 via the communication unit 52.

The detection storage unit 54 of the detection device 50 includes a vehicle data storage unit 54a, a user data storage unit 54b, an abnormality detection data storage unit 54c, and a medical information data storage unit 54d in terms of functional concept.

The vehicle data storage unit 54a is a storage area for storing the vehicle data D1 in the detection storage unit 54. The vehicle data storage unit 54a stores the vehicle data D1 acquired from the integration server 40. The vehicle data storage unit 54a stores the vehicle data D1 including data related to the vehicle identification information D10, the DCM information D11, the vehicle basic information D12, the traveling information D13, and the like transmitted by the data processing device 20.

The user data storage unit 54b is a storage area for storing the user data D2 in the detection storage unit 54. For example, the user data storage unit 54b acquires, from the integration server 40, the user data D2 including the data related to the user basic information D20, the user attribute information D21, the agreement history information D22, the service setting information D23, the contract package information D24, the favorite information D25, and the like registered via the in-vehicle device 10, the user terminal 60, or the like, or automatically generated by the processing unit 44 of the integration server 40, and stores the user data D2.

The abnormality detection data storage unit 54c is a storage area for storing the abnormality detection data D4 in the detection storage unit 54. The abnormality detection data storage unit 54c stores the abnormality detection data D4 generated in the detection device 50. The abnormality detection data storage unit 54c stores the abnormality detection data D4 including data related to the abnormality detection information D41 and the like.

The medical information data storage unit 54d is a storage area for storing the medical information data D5 in the detection storage unit 54. The medical information data storage unit 54d stores the medical information data D5 received from the medical institution terminal 70. The medical information data storage unit 54d stores, for example, the medical information data D5 including data related to the determination result information D51, the treatment program information D52, the consultation recommendation information D53, and the like.

The processing unit 55 of the detection device 50 further includes a detection processing unit 55d in addition to the HMI processing unit 55a, the data processing unit 55b, and the communication processing unit 55c described above in terms of functional concept.

The detection processing unit 55d is a unit that performs a process of detecting the abnormal driving of the user from the vehicle data D1 stored in the vehicle data storage unit 54a according to a predetermined cycle or an operation on the operation input device constituting the HMI unit 51. The detection processing unit 55d detects a specific driving behavior, such as sudden braking, sudden steering, and overlooking of a temporary stop sign, as the abnormal driving from the vehicle data D1. The detection processing unit 55d may detect, as the abnormal driving, a case where the inter-vehicle distance to the preceding vehicle is shorter than an appropriate inter-vehicle distance from the vehicle data D1. Here, the appropriate inter-vehicle distance to the preceding vehicle is, for example, a distance that the vehicle V passes three seconds after the preceding vehicle passes a certain location. When detecting the abnormal driving of the user, the detection processing unit 55d generates information related to the content or the like of the detected abnormal driving as the abnormality detection data D4. Here, the detection processing unit 55d generates the abnormality detection data D4 including, as information related to the content or the like of the detected abnormal driving, information related to the surrounding situation of the vehicle V in a case where the sudden braking, sudden steering, and overlooking of a temporary stop sign occurs, the state of the user captured by the in-vehicle camera during driving, and the biological information of the user.

The HMI processing unit 55a of the detection device 50 is capable of executing a process of controlling the information output device constituting the HMI unit 51, and outputting the abnormality detection data D4 generated by the detection processing unit 55d or the medical information data D5 received from the medical institution terminal 70 via the information output device. The data processing unit 55b of the detection device 50 is capable of executing a process of storing the abnormality detection data D4 generated by the detection processing unit 55d in the abnormality detection data storage unit 54c.

The communication processing unit 55c of the detection device 50 is configured to execute a process of acquiring the vehicle data D1 and the user data D2 from the integration server 40 via the communication unit 52. The communication processing unit 55c is configured to store the vehicle data D1 acquired from the integration server 40 in the vehicle data storage unit 54a, and store the user data D2 acquired from the integration server 40 in the user data storage unit 54b. The communication processing unit 55c is configured to execute a process of acquiring the medical information data D5 from the medical institution terminal 70 via the communication unit 52. The communication processing unit 55c is configured to store the medical information data D5 acquired from the medical institution terminal 70 in the medical information data storage unit 54d. The communication processing unit 55c is configured to transmit the user data D2 and the abnormality detection data D4 in association with each other to the medical institution terminal 70.

### <Basic Configuration of User Terminal>

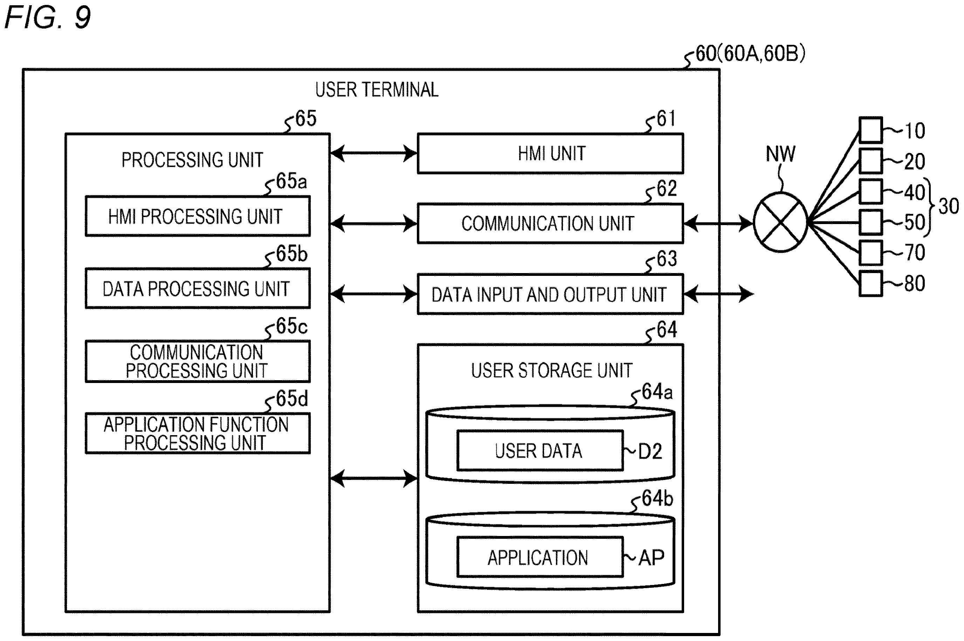

As described above, the user terminal 60 shown in FIG. 9 is a terminal device configured to receive the medical information notification from the detection device 50. The user terminal 60 includes the first user terminal 60A that is mainly owned and used by the user, and the second user terminal 60B that is mainly owned and used by a family of the user, a related person, or the like. Here, there is no substantial difference in configuration between the first user terminal 60A and the second user terminal 60B. The user terminal 60 may be configured to receive various services (for example, the contract management, the parking position search, the vehicle remote control, the emergency notification support, the trouble support, the security notification, and the driving history evaluation) based on data stored in the integration server 40.

Specifically, the user terminal 60 includes an HMI unit 61, a communication unit 62, a data input and output unit 63, a user storage unit 64, and a processing unit 65. The HMI unit 61, the communication unit 62, the data input and output unit 63, the user storage unit 64, and the processing unit 65 are communicably connected to each other.

The HMI unit 61 is a device that performs various inputs and outputs in the user terminal 60. Similarly to the HMI unit 12 described above, the HMI unit 61 includes an operation input device serving as an input unit that performs various inputs to the user terminal 60, and an information output device serving as an output unit that performs various outputs from the user terminal 60.

The communication unit 62 is a communication module capable of communicating with other devices. The communication unit 62 has substantially the same configuration as the communication unit 41 described above, is communicably connected to the network NW regardless of wireless communication or wired communication, and communicates with other devices (for example, the data processing device 20, the integration server 40, the detection device 50, and the medical institution terminal 70) via the network NW.

The data input and output unit 63 is a data input and output device that inputs and outputs data (information) to and from the user terminal 60. The data input and output unit 63 has substantially the same configuration as the data input and output unit 14 described above, and is implemented by a recording medium interface or the like.

The user storage unit 64 is a storage circuit that stores various kinds of data. The user storage unit 64 has substantially the same configuration as the in-vehicle storage unit 15 described above, and is implemented by, for example, an HDD, an SSD, an optical disk, and a semiconductor memory. The user storage unit 64 stores, for example, a program that enables units of the user terminal 60 to implement various functions. The user storage unit 64 stores various kinds of data necessary for various kinds of processes in the processing unit 65, and the processing unit 65 or the like reads the various kinds of data as necessary.

The processing unit 65 is a processing circuit that integrally controls the user terminal 60 and implements various processing functions of the user terminal 60. The processing unit 65 has substantially the same configuration as the processing unit 16 described above, and is implemented by, for example, a processor, a RAM, and a ROM. The processing unit 65 implements processing functions by reading and executing the program stored in the user storage unit 64, for example. For example, the processing unit 65 is capable of executing a process of operating the HMI unit 61, a process of performing data communication via the communication unit 62, and the like.

The processing unit 65 according to the present embodiment includes an HMI processing unit 65a, a data processing unit 65b, and a communication processing unit 65c in order to implement various processing functions of the user terminal 60. The processing unit 65 implements processing functions of the HMI processing unit 65a, the data processing unit 65b, and the communication processing unit 65c, for example, by reading and executing the programs stored in the user storage unit 64.

Similarly to the HMI processing unit 16b described above, the HMI processing unit 65a is a unit having a function capable of executing a process of controlling the HMI unit 61 and operating the HMI unit 61. The HMI processing unit 65a is capable of executing a process of controlling the operation input device constituting the HMI unit 61 and inputting the operation via the operation input device. The HMI processing unit 65a is capable of executing a process of controlling the information output device constituting the HMI unit 61 and outputting information via the information output device.

Similarly to the data processing unit 44b described above, the data processing unit 65b is a unit having a function capable of executing a process related to various kinds of data in the user terminal 60. The data processing unit 65b is capable of executing a process of storing data received via the communication unit 62 in the user storage unit 64. The data processing unit 65b is capable of executing a process of reading data transmitted via the communication unit 62 from the user storage unit 64. The data processing unit 65b is also capable of executing a process of storing data input via the data input and output unit 63 in the user storage unit 64 and a process of reading data output via the data input and output unit 63 from the user storage unit 64. The data processing unit 65b is also capable of executing a process of storing, in the user storage unit 64, data registered in accordance with an operation on the operation input device constituting the HMI unit 61 and a process of reading the data from the user storage unit 64.

Similarly to the communication processing unit 44a described above, the communication processing unit 65c is a unit having a function capable of executing a process of controlling the communication unit 62 and performing data communication. The communication processing unit 65c is capable of executing a process of transmitting and receiving data to and from other devices such as the data processing device 20, the integration server 40, the detection device 50, and the medical institution terminal 70 via the communication unit 62.

The user storage unit 64 of the user terminal 60 includes a user data storage unit 64a and an application storage unit 64b in terms of functional concept.

The user data storage unit 64a is a storage area for storing the user data D2 to be transmitted to the integration server 40 in the user storage unit 64. The user data storage unit 64a stores, for example, the user data D2 including data related to the user basic information D20, the service setting information D23, the favorite information D25, and the like registered in accordance with an operation of the user on the operation input device constituting the HMI unit 61 of the first user terminal 60A.

The application storage unit 64b is a storage area for storing an application program AP used in the user terminal 60 in the user storage unit 64. The application program AP stored in the application storage unit 64b is executed in the user terminal 60 to receive the medical information notification transmitted from the detection device 50 and output the content of the medical information notification to the output unit of the HMI unit 61. Specifically, the HMI unit 61 displays, for example, a determination result related to the cognitive function of the user, the treatment program, and information related to a notification of the consultation recommendation on the display unit. Here, in the user terminal 60, the output unit of the HMI unit 61 includes a display unit having a touch panel function capable of inputting and outputting an operation by the user. In the present embodiment, the notification of the consultation recommendation includes presentation of a behavior recommended to the user. When there is the notification of the consultation recommendation, the output unit of the HMI unit 61 presents, to the user, a behavior recommended to the user. Specifically, in response to receiving the notification of the consultation recommendation, the application program AP is executed in the user terminal 60, and the user terminal 60 displays the behavior recommended to the user according to the content of the medical information notification on the display unit of the HMI unit 61. According to this configuration, the behavior recommended to the user is presented to the user. When the input unit of the HMI unit 61 is operated and an instruction to execute the behavior recommended to the user is received, the user terminal 60 can operate the application program AP to execute the behavior recommended to the user. The application program AP is not limited to one that executes a process corresponding to the notification of the consultation recommendation, and may be capable of executing application functions corresponding to various services linked with the integration server 40.

The processing unit 65 of the user terminal 60 includes the HMI processing unit 65a, the data processing unit 65b, the communication processing unit 65c, and an application function processing unit 65d in terms of functional concept.

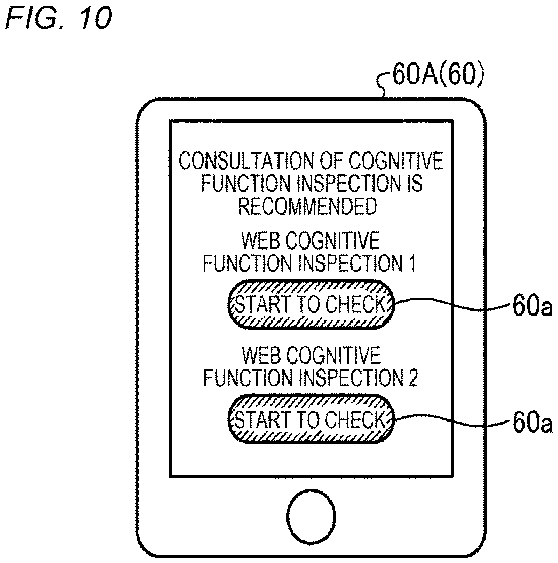

The HMI processing unit 65a of the user terminal 60 is capable of executing a process of controlling the display unit constituting the HMI unit 61, and displaying information and data related to various services executed by the application program AP on the display unit. Each of FIGS. 10 to 12 shows an example of the behavior recommended to the user presented on the display unit when the user terminal 60 displays the notification of the consultation recommendation included in the medical information notification on the display unit of the HMI unit 61.

For example, as shown in FIG. 10, the display unit of the HMI unit 61 displays a message "we recommend you to consult a cognitive function inspection" as the behavior recommended to the user, and displays an icon 60a for starting the cognitive function inspection as the input unit of the HMI unit 61 in order for the user to execute the behavior. The user can consult the cognitive function inspection by touching this icon 60a. A plurality of behaviors recommended to the user may be displayed on the display unit of the HMI unit 61. In the example shown in FIG. 10, two kinds of WEB cognitive function inspections (WEB cognitive function inspection 1 and WEB cognitive function inspection 2) having different contents are selectably displayed for the user.

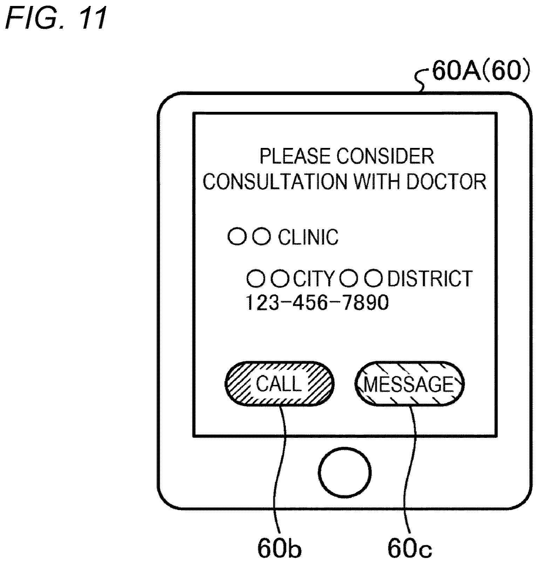

For example, as shown in FIG. 11, the display unit of the HMI unit 61 may display a message "please consider a consultation with a doctor" as the behavior recommended to the user, and display a call icon 60b that allows the user to make a call to the doctor, and a send icon 60c that allows the user to send a message to the doctor. In this case, the user terminal 60 can make a phone call to the doctor when receiving a touch operation on the call icon 60b from the user. The user terminal 60 can send a message to the doctor in response to receiving a touch operation on the send icon 60c from the user.

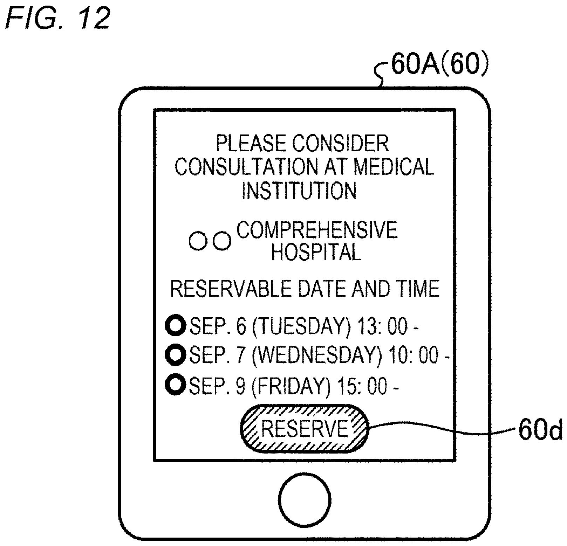

For example, as shown in FIG. 12, the display unit of the HMI unit 61 may display a massage "please consider a consultation at a medical institution" as the behavior recommended to the user, and display a reservation icon 60d for making a reservation for consultation in the medical institution. In this case, the user terminal 60 can make a reservation for consultation in the medical institution in response to receiving a touch operation on the reservation icon 60d from the user.

The first user terminal 60A owned by the user himself or herself and the second user terminal 60B owned by the family of the user, the related person, or the like may be configured to display the same content, or may be configured to display different contents between the content for the user himself or herself and the content for the family, the related person, or the like. For example, when the notification of the consultation recommendation including the determination result and the treatment program related to the cognitive function of the user is issued, the same content may be displayed on the first user terminal 60A and the second user terminal 60B, and the family of the user, the related person, or the like may also know the determination result and the treatment program related to the cognitive function of the user. When different contents between the content for the user himself or herself and the content for the family member, the related person, or the like are notified, the notification of the consultation recommendation as shown in FIGS. 10 to 12 described above may be issued on the first user terminal 60A as the content for the user himself or herself, and a notification related to a simple contact indicating that the user is notified of the consultation recommendation to the medical institution may be issued to the second user terminal 60B as the content for the family member, the related person, or the like.

The data processing unit 65b of the user terminal 60 is capable of executing a process related to a behavior recommended to the user presented on a display unit of the user terminal 60 in accordance with an operation on the operation input device constituting the HMI unit 61. The data processing unit 65b of the user terminal 60 is capable of executing a process of storing, in the user data storage unit 64a as the user data D2, data related to the user basic information D20, the service setting information D23, the favorite information D25, and the like registered in accordance with an operation on the operation input device constituting the HMI unit 61. The data processing unit 65b of the user terminal 60 is capable of executing a process of reading the user data D2 stored in the user data storage unit 64a in response to a request.

The communication processing unit 65c of the user terminal 60 is capable of executing a process of transmitting the user data D2 stored in the user data storage unit 64a to the integration server 40 via the communication unit 62. The communication processing unit 65c of the user terminal 60 is capable of executing a process of transmitting various commands and requests to the integration server 40 and the like via the communication unit 62. The communication processing unit 65c of the user terminal 60 is capable of executing a process of receiving the various commands and requests from the integration server 40, the detection device 50, and the like via the communication unit 62. The communication processing unit 65c of the user terminal 60 is capable of executing a process of receiving various kinds of information according to a service content and an application function from the integration server 40, the detection device 50, or the like via the communication unit 62. The data received by the communication processing unit 65c of the user terminal 60 from the integration server 40, the detection device 50, or the like according to the service content or the application function may include, for example, the vehicle data D1, the user data D2, the abnormality detection data D4, and the medical information data D5 stored in the integration server 40, and various kinds of information, data, and the like generated based on the data.

The application function processing unit 65d is a unit having a function capable of executing a process of executing the application program AP stored in the application storage unit 64b. The application function processing unit 65d activates the application program AP stored in the application storage unit 64b in response to, for example, receiving the notification of the consultation recommendation from the detection device 50 or an operation performed on the operation input device constituting the HMI unit 61, and is capable of executing application functions corresponding to various services.

### <Basic Configuration of Medical Institution Terminal>

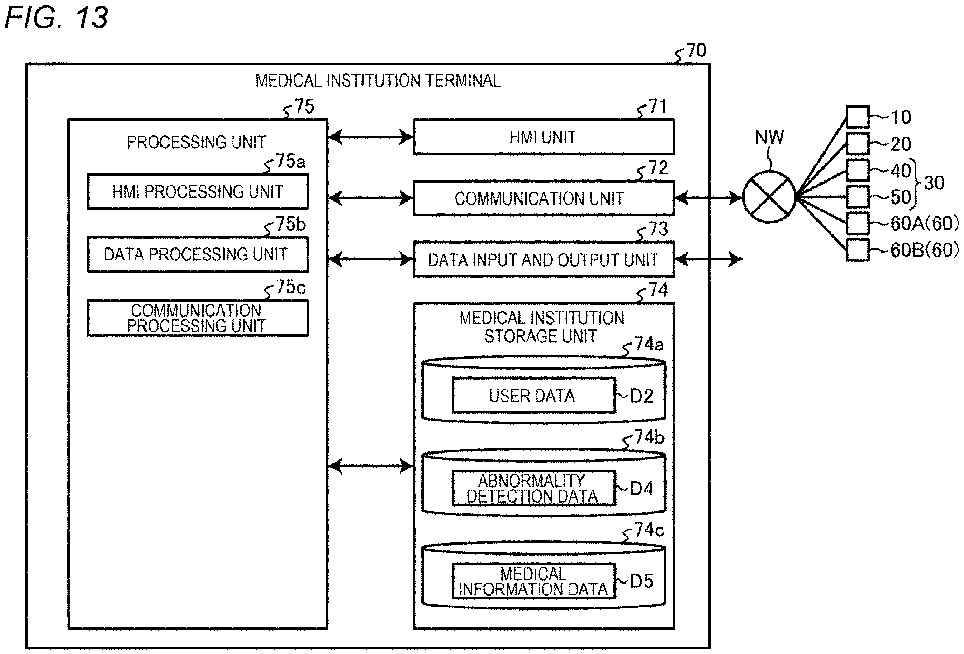

As described above, the medical institution terminal 70 shown in FIG. 13 is configured to communicate with the detection device 50, and is a device mainly used by the medical worker such as a doctor. Typically, the medical institution terminal 70 is a terminal device mainly provided at a medical institution. The medical institution terminal 70 is configured to receive the user data D2 and the abnormality detection data D4 transmitted from the detection device 50 and store the data in the storage unit. The medical institution terminal 70 is configured such that the medical worker such as a doctor can confirm the contents of the user data D2 and the abnormality detection data D4. The medical institution terminal 70 is configured to store, in the storage unit, the medical information data D5 created by a doctor of the medical institution or the like based on the abnormality detection data D4 and the opinion of the medical worker, and transmit the medical information data D5 to the detection device 50. The medical institution terminal 70 may be configured to access a server, a storage terminal, or the like in which diagnosis information such as an electronic medical record is stored. In this case, the medical institution terminal 70 is configured to specify the diagnosis information of the user in the medical institution from the user data D2. When the diagnosis information of the user in the medical institution can be specified from the user data D2, the medical worker can create the medical information data D5 in consideration of the content of the diagnosis information of the user in addition to the abnormality detection data D4. The user data D2 stored in the medical institution terminal 70 includes information that can be collated with diagnosis information of a patient stored in the medical institution. For example, the medical institution terminal 70 collates a personal number or a medical insurance card number included in the user data D2 with a personal number or a medical insurance card number included in the diagnosis information of the user in the medical institution to specify the diagnosis information of the target user. The diagnosis information of the target user may be specified by utilizing information such as a name, an address, a telephone number, an age, and a gender of the user included in the user data D2. The medical institution terminal 70 may transmit the medical information data D5 to a storage device such as a server or a storage terminal provided in the medical institution, and store the medical information data D5 in association with the diagnosis information (electronic medical record or the like) of the user stored in the storage device.

The medical institution terminal 70 includes an HMI unit 71, a communication unit 72, a data input and output unit 73, a medical institution storage unit 74, and a processing unit 75. The HMI unit 71, the communication unit 72, the data input and output unit 73, the medical institution storage unit 74, and the processing unit 75 are communicably connected to each other.

The HMI unit 71 is a device that performs various inputs and outputs in the medical institution terminal 70. Similarly to the HMI unit 12 described above, the HMI unit 71 includes an operation input device that performs various inputs to the medical institution terminal 70 and an information output device that performs various outputs from the medical institution terminal 70.

The communication unit 72 is a communication module capable of communicating with devices other than the medical institution terminal 70. The communication unit 72 has substantially the same configuration as the communication unit 21 described above, is communicably connected to the network NW regardless of whether the communication is wireless communication or wired communication, and communicates with other devices (for example, the service providing device 30, the integration server 40, and the detection device 50) via the network NW.

The data input and output unit 73 is a data input and output device that inputs and outputs data (information) to and from the medical institution terminal 70. The medical institution terminal 70 has substantially the same configuration as the data input and output unit 14 described above, and is implemented by a recording medium interface or the like.

The processing unit 75 of the medical institution terminal 70 includes an HMI processing unit 75a, a data processing unit 75b, and a communication processing unit 75c in terms of functional concept.

Similarly to the HMI processing unit 16b described above, the HMI processing unit 75a of the medical institution terminal 70 is a unit having a function capable of executing a process of controlling the HMI unit 71 and operating the HMI unit 71. The HMI processing unit 75a is capable of executing a process of controlling the operation input device constituting the HMI unit 71 and inputting an operation via the operation input device. The HMI processing unit 75a is capable of executing a process of controlling the information output device constituting the HMI unit 71 and outputting information via the information output device.

Similarly to the data processing unit 44b described above, the data processing unit 75b of the medical institution terminal 70 is a unit having a function capable of executing a process related to various kinds of data in the user terminal 60. The data processing unit 75b is capable of executing a process of storing data received via the communication unit 72 in the medical institution storage unit 74. The data processing unit 75b is capable of executing a process of reading the data transmitted via the communication unit 72 from the medical institution storage unit 74. The data processing unit 75b is also capable of executing a process of storing data input via the data input and output unit 73 in the medical institution storage unit 74, and a process of reading data output via the data input and output unit 73 from the medical institution storage unit 74. The data processing unit 75b is also capable of executing a process of storing, in the medical institution storage unit 74, data created according to an operation on the operation input device constituting the HMI unit 51, and a process of reading the data from the medical institution storage unit 74.

The communication processing unit 75c of the medical institution terminal 70 is capable of executing a process of receiving the user data D2 and the abnormality detection data D4 from the detection device 50 via the communication unit 72. The communication processing unit 75c is capable of executing a process of transmitting the medical information data D5 to the detection device 50 via the communication unit 72.

Similarly to the communication processing unit 44a described above, the communication processing unit 75c of the medical institution terminal 70 is a unit having a function of executing a process of controlling the communication unit 72 and performing the data communication. The communication processing unit 75c is capable of executing a process of transmitting and receiving data to and from other devices such as the data processing device 20, the detection device 50, and the user terminal 60 via the communication unit 72.

The medical institution storage unit 74 includes a user data storage unit 74a, an abnormality detection data storage unit 74b, and a medical information data storage unit 74c in terms of functional concept.

The user data storage unit 74a of the medical institution terminal 70 is a storage area for storing the user data D2 in the medical institution terminal 70. The user data storage unit 74a stores the user data D2 transmitted from the detection device 50 in association with the abnormality detection data D4. The abnormality detection data storage unit 74b of the medical institution terminal 70 is a storage area for storing the abnormality detection data D4 in the medical institution terminal 70. The abnormality detection data storage unit 74b stores the abnormality detection data D4 transmitted from the detection device 50 in association with the user data D2. The medical information data storage unit 74c is a storage area for storing the medical information data D5. The medical information data storage unit 74c stores, as the medical information data D5, data including the determination result related to the cognitive function of the user, the treatment program, the information related to the consultation recommendation on the disease related to the cognitive function according to the operation of the operation input device constituting the HMI unit 71, and the like.

The data processing unit 75b receives the abnormality detection data D4 associated with the user data D2 from the detection device 50 via the communication unit 72, stores the user data D2 in the user data storage unit 74a while maintaining a state in which the user data D2 and the abnormality detection data D4 are associated with each other, and stores the abnormality detection data D4 in the abnormality detection data storage unit 74b. By holding the association between the user data D2 and the abnormality detection data D4, the user corresponding to the abnormality detection data D4 can be specified in the medical institution.

An outline of the overall configuration of the abnormality detection system 1 according to the present embodiment has been described above.

### <Flow of Data in Abnormality Detection System>

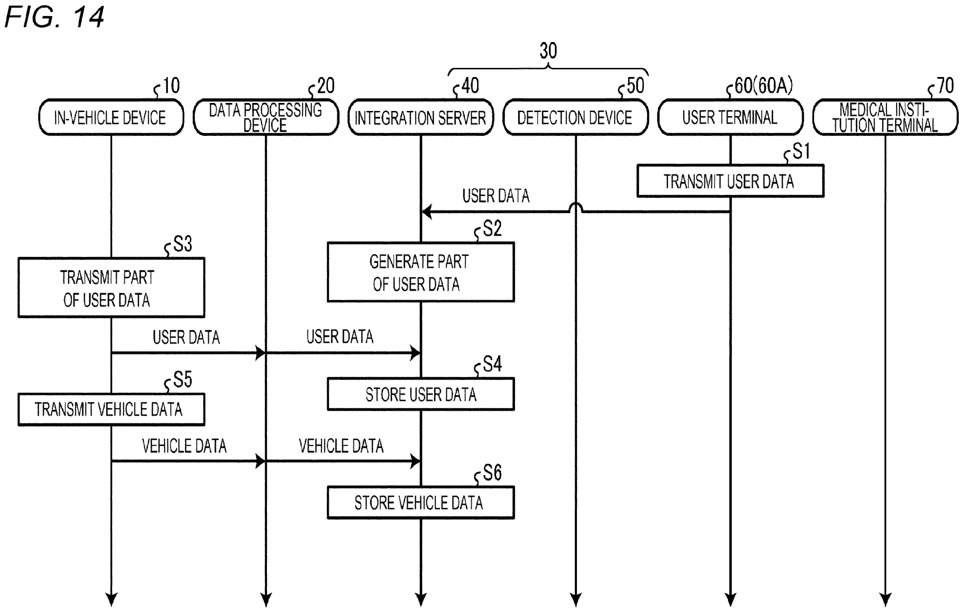

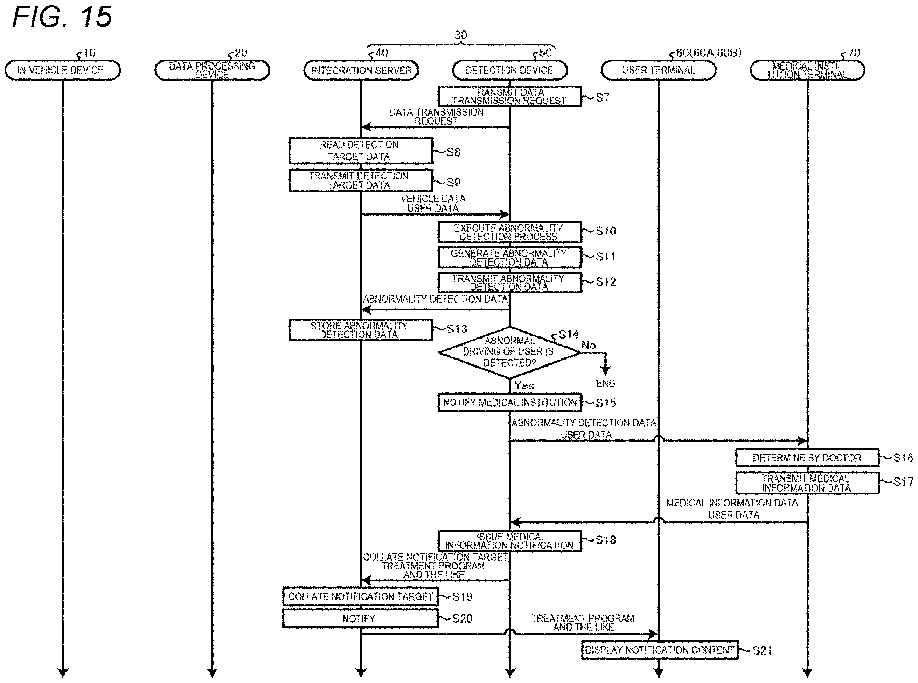

Next, an example of a flow of each process in the abnormality detection system 1 according to the present embodiment will be described with reference to FIGS. 14 and 15. In the following description, transmission and reception of data are performed via the network NW, but the description thereof may be omitted. In the following description, in order to mainly focus on the flow of data, a description of specific processing operations related to the processing unit and the like in each device may be omitted as appropriate, and the outline of the entire configuration described above will be referred to as necessary. The flow of each process described below is merely an example of a representative process, and the present invention is not limited thereto.

### <Storage of User Data and Vehicle Data>

First, as shown in FIG. 14, the user terminal 60 (the first user terminal 60A) transmits, as the user data D2 to the integration server 40, data related to the user basic information D20, the service setting information D23, the favorite information D25, and the like registered in the user terminal 60 (the first user terminal 60A) in the user data D2 (step S1). The user terminal 60 (the first user terminal 60A) is capable of transmitting not only the initially registered user data D2 but also the user data D2 updated after the initial registration to the integration server 40. The user terminal 60 (the first user terminal 60A) can transmit, for example, the user basic information D20 including the terminal registration information of the second user terminal 60B as the user data D2 such that the medical information notification for the user is also issued to the second user terminal 60B used by the family of the user, the related person, or the like. In a case where the user newly registers a service related to the notification of the consultation recommendation, in a case where the registration information is changed for the service related to the notification of the consultation recommendation that has already been registered, or the like, it is possible to register the vehicle identification information D10 in the user data D2 by including the vehicle identification information D10 in the terminal registration information using the user terminal 60 (the first user terminal 60A). Accordingly, even when the vehicle data D1 is not stored in the integration server 40, the user data D2 and the abnormality detection data D4 can be managed in association with each other based on the vehicle identification information D10.

In response to receiving the user data D2 from the user terminal 60 (the first user terminal 60A), the integration server 40 automatically generates a part of the user data D2 based on the received user data D2 (step S2). Here, the integration server 40 automatically generates data related to the user attribute information D21, the agreement history information D22, the contract package information D24, and the like based on data related to the user basic information D20, the service setting information D23, the favorite information D25, and the like received from the user terminal 60, for example.

The user data D2 may be registered, for example, via the in-vehicle device 10. In this case, the in-vehicle device 10 can register a part of the information of the user data D2 in accordance with an operation on the HMI unit 12 performed by the user, transmits data related to the registered part of the information as the user data D2 to the data processing device 20, and transmits the data to the integration server 40 via the data processing device 20 (step S3).

The integration server 40 stores the received user data D2 and the automatically generated user data D2 in the server storage unit 43 (user data storage unit 43c) in association with the user ID D60 (step S4).

The in-vehicle device 10 transmits the vehicle data D1 detected and acquired by the detection and acquisition unit 11 to the data processing device 20 at an appropriate timing (step S5). In this case, the vehicle data D1 includes the vehicle identification information D10. In response to receiving the vehicle data D1 from the in-vehicle device 10, the data processing device 20 transmits the received vehicle data D1 to the integration server 40.

In response to receiving the vehicle data D1 from the data processing device 20, the integration server 40 stores the vehicle data D1 in the server storage unit 43 (the vehicle data storage unit 43a) in association with the user ID D60 and the vehicle ID D61 based on the ID data D6. In this case, since the user ID D60 and the vehicle ID D61 are associated with each other, the vehicle data D1 can be associated with the user data D2 and stored in the server storage unit 43 (the user data storage unit 43c) based on the vehicle identification information D10 (step S6).

### <Detection of Abnormal Driving of User and Medical Information Notification>

As shown in FIG. 15, when the vehicle data D1 used for detecting the abnormal driving of the user is acquired from the integration server 40, the detection device 50 transmits a data transmission request to the integration server 40 (step S7). In response to receiving the data transmission request from the detection device 50, the integration server 40 reads the target vehicle data D1 and the user data D2 corresponding to the vehicle data D1 (step S8). Then, the integration server 40 transmits the read vehicle data D1 and the user data D2 corresponding to the vehicle data D1 to the detection device 50 (step S9). Here, the integration server 40 transmits the vehicle data D1 and the user data D2 in association with each other to the detection device 50. In response to receiving the vehicle data D1 and the user data D2, the detection device 50 detects the abnormal driving of the user in the vehicle V from the vehicle data D1 (step S10). Then, the detection device 50 generates the abnormality detection data D4 including the content of the detection result, and stores the abnormality detection data D4 in the detection storage unit 54 (step S11). Here, the detection device 50 stores the abnormality detection data D4 in the detection storage unit 54 in association with the vehicle data D1 and the user data D2. Thereafter, the detection device 50 transmits the generated abnormality detection data D4 to the integration server 40 (step S13). Here, when the detection device 50 transmits the abnormality detection data D4 to the integration server 40, the integration server 40 collates the abnormality detection data D4 received from the detection device 50 with the user data D2 or the like stored in the integration server 40, and transmits the data in a manner of being able to store in association with each other. Specifically, for example, the detection device 50 transmits the abnormality detection data D4 to the integration server 40 in association with the vehicle data D1 and the user data D2. Then, the integration server 40 collates the abnormality detection data D4 received from the detection device 50 based on the vehicle identification information D10 included in the vehicle data D1, the user data D2, and the like stored in the integration server 40, stores the data in the server storage unit 43 in association with each other, and manages the data (step S13).

The detection device 50 confirms whether the abnormal driving of the user is detected based on the abnormality detection data D4 (step S14). Here, if the detection device 50 confirms that there is no abnormality in driving of the user in the vehicle V based on the abnormality detection data D4, the process is ended until a next data transmission request is transmitted. If the detection device 50 confirms that there is an abnormality in driving of the user based on the abnormality detection data D4 (if the abnormal driving of the user is detected), the detection device 50 transmits the user data D2 and the abnormality detection data D4 in association with each other to the medical institution terminal 70 of the medical institution (step S15). In this case, the detection device 50 may perform, with respect to the user, an agreement confirmation for transmitting the user data D2 and the abnormality detection data D4 to the medical institution terminal 70 of the medical institution. When performing the agreement confirmation, the detection device 50 specifies the user terminal 60 (the first user terminal 60A) of the user that performs the agreement confirmation based on the user data D2, and notifies the user terminal 60 (the first user terminal 60A) of the agreement confirmation. In response to an operation from the user, the user terminal 60 (the first user terminal 60A) transmits, to the detection device 50, a response indicating whether to agree to transmit the user data D2 and the abnormality detection data D4 to the medical institution terminal 70 of the medical institution. The detection device 50 transmits the user data D2 and the abnormality detection data D4 to the medical institution terminal 70 of the medical institution only when the user agrees to transmit the user data D2 and the abnormality detection data D4 to the medical institution terminal 70 of the medical institution.

In response to receiving the user data D2 and the abnormality detection data D4 transmitted from the detection device 50, the medical institution terminal 70 stores the user data D2 and the abnormality detection data D4 in association with each other in the medical institution storage unit 74 and manages the data. The medical institution terminal 70 processes the user data D2 and the abnormality detection data D4 to be utilizable by the medical worker such as a doctor in the medical institution. The processed user data D2 and the abnormality detection data D4 are utilized by the medical worker in the medical institution to determine the presence or absence of the indication or the symptom of the disease related to the cognitive function of the user. The medical worker such as a doctor in the medical institution determines the presence or absence of the indication or the symptom of the disease related to the cognitive function of the user based on the abnormality detection data D4, the opinion of the medical worker himself or herself, and the like (step S16). Then, the medical worker such as a doctor in the medical institution creates the medical information data D5 based on the determination result, and stores the medical information data D5 in the medical institution storage unit 74 of the medical institution terminal 70. Here, the medical institution terminal 70 stores the medical information data D5 in association with the user data D2 and the abnormality detection data D4 in the medical institution storage unit 74. The medical institution terminal 70 transmits the medical information data D5 to the detection device 50 (step S17). Here, when the medical institution terminal 70 transmits the medical information data D5 to the detection device 50, the detection device 50 collates the medical information data D5 received from the medical institution terminal 70 with the user data D2 or the like stored in the detection device 50, and transmits the data in a manner of being able to be stored in association with each other. Specifically, for example, the medical institution terminal 70 transmits the medical information data D5 to the detection device 50 in association with the user data D2 corresponding to the medical information data D5. Then, the detection device 50 collates the medical information data D5 received from the medical institution terminal 70 with the user data D2 or the like stored in the detection device 50, stores the data in the detection storage unit 54 in association with each other, and manages the data.

The detection device 50 transmits the medical information including the content based on the medical information data D5 to the integration server 40 and issues a medical information notification (step S18). In response to receiving the medical information from the detection device 50, the integration server 40 performs collation on a target to be notified of the medical information (step S19). By collating the notification target, the user terminal 60 of the user to be notified of the medical information is specified. Here, when issuing the medical information notification to the user terminal 60 via the integration server 40, the detection device 50 transmits, to the integration server 40, the medical information including the treatment program and the like based on the medical information data D5 in a state in which the integration server 40 can specify the user terminal 60 to which the medical information notification is issued. For example, the detection device 50 transmits the user data D2 and the medical information including the treatment program and the like in association with each other to the integration server 40, so that the integration server 40 can specify the user terminal 60 of the user to be notified of the medical information based on the user data D2 received from the detection device 50. For example, the detection device 50 transmits the vehicle identification information D10 included in the vehicle data D1 and the medical information including the treatment program and the like to the integration server 40 in association with each other, so that the integration server 40 can specify the user terminal 60 of the user to be notified of the medical information based on the vehicle identification information D10 received from the detection device 50.

When specifying the user terminal 60 to be notified of the medical information, the integration server 40 transmits the medical information notification to the specified user terminal 60 (step S20). In response to receiving the medical information notification from the integration server 40, the user terminal 60 displays the content corresponding to the medical information notification on the display unit (step S21). For example, when the content of the medical information notification is a determination result related to the cognitive function of the user, information related to the presence or absence, the content, and the like of the indication or the symptom of the disease related to the cognitive function of the user is output to the user terminal 60. When the content of the medical information notification includes the treatment program, information related to the treatment program determined to be suitable for the user by the doctor of the medical institution is output to the user terminal 60. When the content of the medical information notification includes the consultation recommendation on the disease related to the cognitive function, the information related to the consultation recommendation as shown in FIGS. 10 to 12 is output.

In the above embodiment, the configuration in which the service providing device 30 in the abnormality detection system 1 includes the integration server 40 (a storage unit) and the detection device 50 (a notification unit and a detection unit) has been described as an example, but the present invention is not limited to this configuration. For example, the service providing device 30 in the abnormality detection system 1 may include at least the notification unit and the detection unit. In this case, the abnormality detection system 1 acquires the vehicle data D1 or the like from an in-vehicle device mounted on the vehicle V, an external storage device (a storage unit), or the like, and the detection unit determines abnormal driving of the user of the vehicle V based on the acquired vehicle data D1 or the like.

### <Effects of First Embodiment>

As described above, the abnormality detection system 1 according to the present embodiment includes: the service providing device 30 including the detection unit that detects the abnormal driving of the user of the vehicle V from the vehicle data D1 including at least the traveling information D13 of the vehicle V and the notification unit that issues the notification related to the medical information to the user; and the user terminals 60, 60A, and 60B configured to communicate with the service providing device 30. The notification unit notifies the user terminals 60, 60A, and 60B of the medical information created based on the information related to the abnormal driving detected by the detection unit and the opinion of the medical worker of the medical institution.

The abnormality detection system 1 according to the present invention detects the abnormal driving of the user from the vehicle data D1 collected by the in-vehicle device 10, and provides the information related to the detected abnormal driving of the user to the medical institution, and thus, the presence or absence of the indication, the symptom, or the like of deterioration in cognitive function can be determined in the medical institution from the driving situation of the vehicle V, which is a daily behavior of the user. Accordingly, it is possible to discover the indication, the symptom, and the like of deterioration in cognitive function of the user at an early stage. In the abnormality detection system 1 according to the present embodiment, the medical worker such as a doctor in the medical institution can determine a disease and the like related to the cognitive function of the user based on the abnormality detection data D4 including the information related to the abnormal driving of the user, and thus, unlike a simple inspection related to the cognitive function in the related art, rather than making a determination based on a temporary state of a person who takes such a simple inspection, it is possible to determine the presence or absence and the like of the disease related to the cognitive function of the user based on the driving situation of the vehicle V, which is a daily behavior of the user. Accordingly, as compared with the simple inspection as described above, since it is possible to determine the indication or the symptom of deterioration in cognitive function at appropriate time, in particular, it is possible to detect an abnormality in the cognitive function at an initial stage.

The abnormality detection system 1 according to the present embodiment includes: the service providing device 30 including the storage unit that stores the user data D2 related to the user of the vehicle V and the vehicle data D1 including the traveling information D13 of the vehicle V, the detection unit that detects abnormal driving of the user in the vehicle V from the vehicle data D1 and stores the abnormality detection data D4 including a content of the detected abnormal driving in the storage unit, and the notification unit that issues a notification related to medical information to the user; and the user terminals 60, 60A, and 60B that communicate with the service providing device 30. The notification unit transmits at least the abnormality detection data D4 to the medical institution terminal 70 of the medical institution, receives, from the medical institution terminal 70, the medical information data D5 created based on the abnormality detection data D4 and an opinion of a medical worker of the medical institution, and notifies the user terminals 60, 60A, and 60B of the medical information including a content based on the medical information data D5.

The abnormality detection system 1 according to the present invention detects the abnormal driving of the user from the vehicle data D1 collected by the in-vehicle device 10 and stored in the storage unit, and provides the information related to the detected abnormal driving of the user to the medical institution, and thus, the presence or absence of the indication, the symptom, or the like of deterioration in cognitive function can be determined in the medical institution from the driving situation of the vehicle V, which is a daily behavior of the user. Accordingly, it is possible to discover the indication, the symptom, and the like of deterioration in cognitive function of the user at an early stage. In the abnormality detection system 1 according to the present embodiment, the medical worker such as a doctor in the medical institution can determine a disease and the like related to the cognitive function of the user based on the abnormality detection data D4 including the information related to the abnormal driving of the user, and thus, unlike a simple inspection related to the cognitive function in the related art, rather than making a determination based on a temporary state of a person who takes such a simple inspection, it is possible to determine the presence or absence and the like of the disease related to the cognitive function of the user based on the driving situation of the vehicle V, which is a daily behavior of the user. Accordingly, as compared with the simple inspection as described above, since it is possible to determine the indication or the symptom of deterioration in cognitive function at appropriate time, in particular, it is possible to detect an abnormality in the cognitive function at an initial stage.

In the abnormality detection system 1 according to the present embodiment, the notification unit receives the medical information data D5 created based on the abnormality detection data D4 and the opinion of the medical worker in the medical institution from the medical institution terminal 70, specifies the user terminals 60, 60A, 60B from the user data D2 corresponding to the medical information data D5, and issues the medical information notification, which is a notification of the content based on the medical information data D5, to the user terminals 60, 60A, 60B.

The abnormality detection system 1 according to the present embodiment notifies the user (the user terminal 60) of the medical information created based on the information related to the abnormal driving of the user and the opinion of the medical worker of the medical institution, and thus the user can be prompted to undergo early treatment regarding the disease related to the cognitive function of the user.

In the abnormality detection system 1 according to the present embodiment, the medical information data D5 includes information related to at least one of a treatment program determined to be suitable for the user by the medical worker, a determination result related to the cognitive function of the user, and a consultation recommendation on the disease related to the cognitive function.

The abnormality detection system 1 according to the present embodiment notifies the user (the user terminal 60) of the treatment program determined to be suitable for the user by the medical worker such as a doctor of the medical institution, thereby allowing the user to know a specific treatment plan or the like and, for example, to improve an intention of the user to consult the medical institution. By notifying the user (the user terminal 60) of the determination result related to the cognitive function of the user, the user can know the cognitive function state of himself or herself and the necessity of a treatment, and the intention of the user to consult with the medical institution can be improved. By issuing the user of the notification of the consultation recommendation on the disease related to the cognitive function, it is possible to improve the intention of the user to consult the medical institution.

In the abnormality detection system 1 according to the present embodiment, in a case where the medical information data D5 includes the information related to the consultation recommendation, the notification unit transmits the medical information including the presentation of the behavior recommended to the user to the user terminals 60, 60A, and 60B, and the user terminals 60, 60A, and 60B present the behavior recommended to the user and display the operation of executing the behavior in a receivable manner.

The abnormality detection system 1 according to the present embodiment presents a specific behavior recommended to the user, and is provided with an input unit (the icons 60a to 60d) capable of receiving an operation of executing the behavior, so that it is possible to improve the intention of the user to visit the medical institution. Deterioration in cognitive function may not appear in an external wound, and for example, the user may not know which department the specialist corresponds to, or may be hard to visit a comprehensive hospital for an initial inspection. Even in such a case, the user terminal 60 displays the input unit (the icons 60a to 60d) in an operable manner, so that the user can smoothly take the recommended behavior. That is, by providing the input unit (the icons 60a to 60d) in the user terminal 60 in an operable manner, it is possible to reduce a psychological barrier when the user makes a reservation for the medical institution or the like.

In the abnormality detection system 1 according to the present embodiment, the storage unit stores the user data D2, the vehicle data D1, and the abnormality detection data D4 in association with each other for each user, and the user data D2 includes information that can be collated with diagnosis information of a patient stored in the medical institution.

The abnormality detection system 1 according to the present embodiment includes the information that can be collated with the diagnosis information of the patient stored in the medical institution in the user data D2, thereby enabling the medical worker such as a doctor of the medical institution to determine the presence or absence and the like of the indication or the symptom of the disease related to the cognitive function while referring to the past and current diagnosis information of the user. Accordingly, the medical information having more detailed contents can be notified to the user (the user terminal 60).

In the abnormality detection system 1 according to the present embodiment, the service providing device 30 includes the integration server 40 that manages the user data D2, the vehicle data D1, and the abnormality detection data D4, and the detection device 50 that communicates with the integration server 40, and the integration server 40 includes a storage unit, and the detection device 50 includes a detection unit and a notification unit.

In the abnormality detection system 1 according to the embodiment, personal information such as the user data D2 and the abnormality detection data D4 is collectively managed by one server (the integration server 40), and thus, various kinds of data are not managed in a distributed manner, and secret retention can be improved. As compared with a case where data management is performed by a plurality of servers, it is possible to reduce data to be redundantly stored and to reduce a necessary storage capacity. Accordingly, it is possible to effectively utilize a storage resource.

The base station device (the service providing device 30) according to the present embodiment includes the service providing device 30 including: the storage unit that stores the user data D2 related to the user of the vehicle V and the vehicle data D1 including the traveling information D13 of the vehicle V; the detection unit that detects abnormal driving of the user in the vehicle V from the vehicle data D1 and stores the abnormality detection data D4 including a content of the detected abnormal driving in the storage unit; and the notification unit that issues a notification related to medical information to the user. The notification unit transmits at least the abnormality detection data D4 to the medical institution terminal 70 of the medical institution, receives, from the medical institution terminal 70, the medical information data D5 created based on the abnormality detection data D4 and an opinion of a medical worker of the medical institution, and notifies the user terminals 60, 60A, and 60B of the medical information including a content based on the medical information data D5.

The base station device (the service providing device 30) according to the present embodiment detects the abnormal driving of the user from the vehicle data D1 collected by the in-vehicle device 10, and provides the information related to the detected abnormal driving of the user to the medical institution, and thus, the presence or absence of the indication, the symptom, or the like of deterioration in cognitive function can be determined in the medical institution from the driving situation of the vehicle V, which is a daily behavior of the user. Accordingly, it is possible to discover the indication, the symptom, and the like of deterioration in cognitive function of the user at an early stage. In the base station device (the service providing device 30) according to the present embodiment, the medical worker such as a doctor in the medical institution can determine the disease and the like related to the cognitive function of the user based on the abnormality detection data D4 including the information related to the abnormal driving of the user, and thus, unlike a simple inspection related to the cognitive function in the related art, rather than making a determination based on a temporary state of a person who takes such a simple inspection, it is possible to determine the presence or absence and the like of the disease related to the cognitive function of the user based on the driving situation of the vehicle V, which is a daily behavior of the user. Accordingly, as compared with the simple inspection as described above, since it is possible to determine the indication or the symptom of deterioration in cognitive function at appropriate time, in particular, it is possible to detect an abnormality in the cognitive function at an initial stage.

### <Second Embodiment>

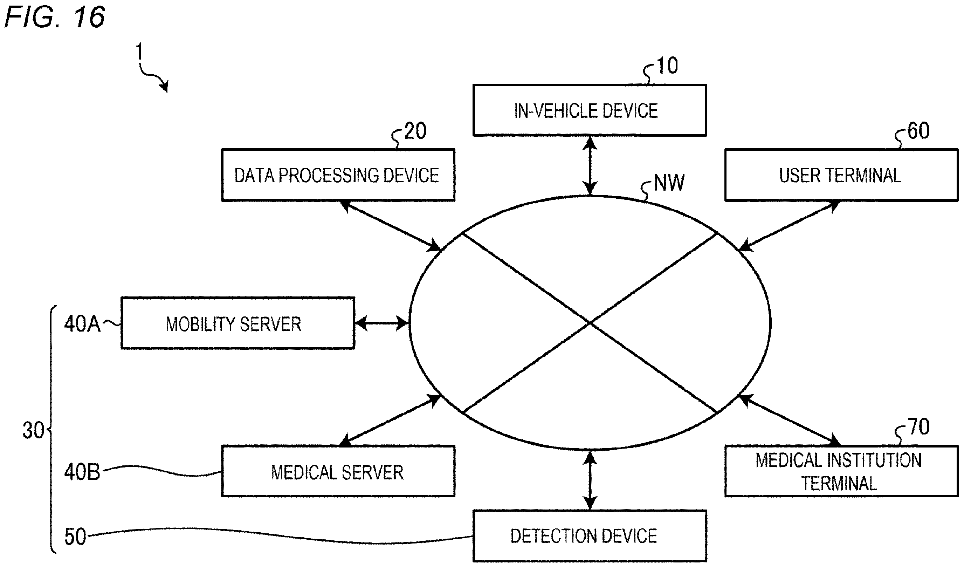

Next, a second embodiment will be described with reference to FIGS. 16 to 21. The abnormality detection system 1 and the base station device according to the present embodiment are different from the abnormality detection system 1 and the base station device according to the embodiment described above in that two servers (a mobility server 40A and a medical server 40B) cooperate with each other to manage various kinds of data instead of the integration server 40. That is, in the abnormality detection system 1 and the base station device according to the present embodiment, the servers (the mobility server 40A and the medical server 40B) configured separately and independently cooperate with each other to manage various kinds of data. The mobility server 40A and the medical server 40B constitute a storage unit of the abnormality detection system 1 in cooperation with each other. The mobility server 40A and the medical server 40B may be implemented by, for example, various computer devices. The mobility server 40A and the medical server 40B can also be implemented by, for example, installing programs for realizing various processes in a known computer device. Here, the mobility server 40A and the medical server 40B may be separately and independently configured as different physical servers, or may be separately and independently configured as so-called virtual servers on the same physical server or a plurality of physical servers according to various virtualization techniques. That is, the mobility server 40A and the medical server 40B are configured to hold data independently of each other. As described above, the mobility server 40A and the medical server 40B may be physically and structurally independent of each other, or may be configured such that, for example, each component such as a processor is physically common and storage areas are independent of each other.

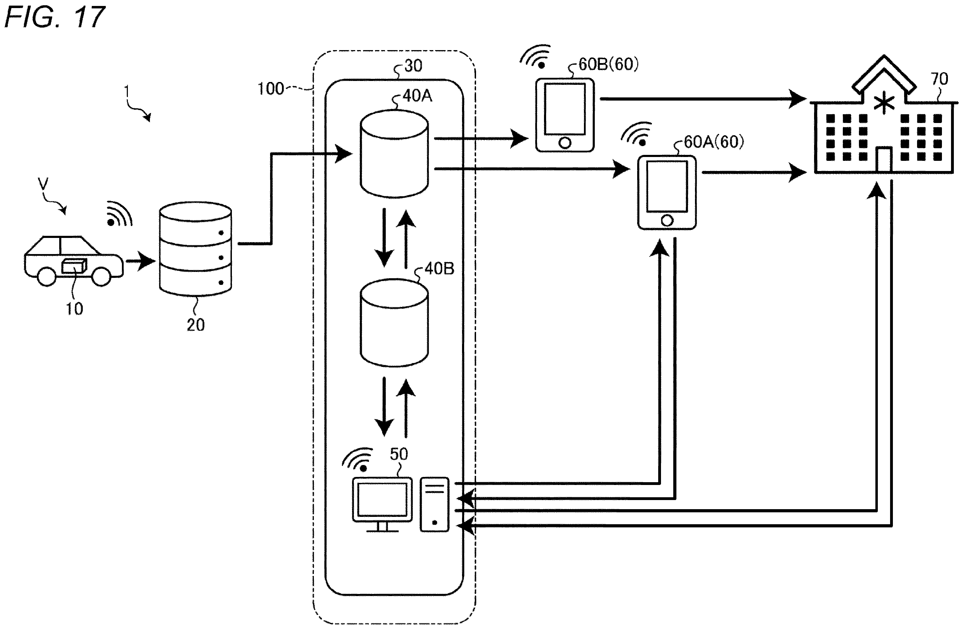

As shown in FIGS. 16 and 17, in the abnormality detection system 1 according to the present embodiment, the mobility server 40A, the medical server 40B, and the detection device 50 constitute the service providing device 30. The in-vehicle device 10, the data processing device 20, the service providing device 30, the user terminal 60, and the medical institution terminal 70 are configured to communicate with each other through the network NW. Here, the service providing device 30 constitutes, for example, the base station device provided in the base station 100 that relays communication between the vehicle V and the user terminal 60 or the medical institution terminal 70, and is managed by, for example, the business operator that develops various services related to medical care or the business operator that develops the vehicle V The detection device 50 does not need to be disposed in the base station 100, and may be managed in a place different from the base station 100. For example, the detection device 50 may be configured to access the base station device from a remote location different from a location of the base station. That is, at least the mobility server 40A and the medical server 40B may constitute the base station device.

As shown in FIG. 17, in the abnormality detection system 1 according to the present embodiment, the vehicle data D1 is transmitted from the in-vehicle device 10 of the vehicle V to the mobility server 40A. Here, the vehicle data D1 is transmitted from the in-vehicle device 10 to the mobility server 40A via the data processing device 20. The mobility server 40A stores the vehicle data D1 received from the in-vehicle device 10.

Configurations of the mobility server 40A and the medical server 40B in the abnormality detection system 1 according to the present embodiment will be described below.

### <Basic Configurations of Mobility Server and Medical Server>

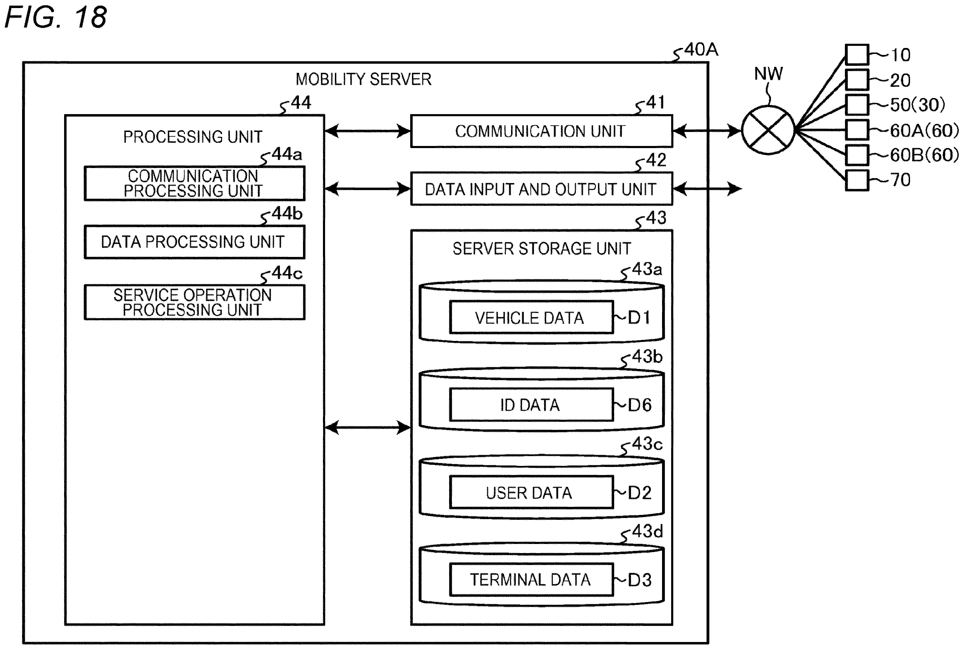

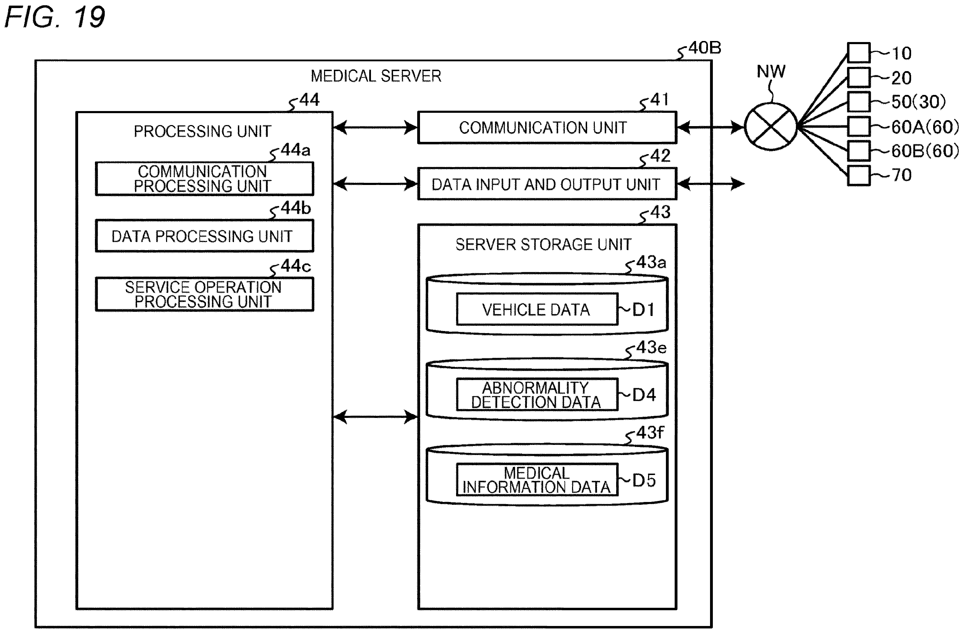

The mobility server 40A shown in FIG. 18 and the medical server 40B shown in FIG. 19 are servers corresponding to the integration server 40 in the abnormality detection system 1 according to the first embodiment described above. In the abnormality detection system 1 according to the present embodiment, the mobility server 40A and the medical server 40B are configured to cooperate to implement the same function as the integration server 40. That is, the mobility server 40A and the medical server 40B function as connected servers that cooperate with each other to deploy connected services. In the abnormality detection system 1 according to the present embodiment, not only services for notifying the medical information, but also connected services such as contract management, parking position search, vehicle remote control, emergency notification support, trouble support, security notification, and driving history evaluation can be provided to the user by using data stored in the mobility server 40A.

As shown in FIGS. 18 and 19, basic configurations of the mobility server 40A and the medical server 40B are the same as that of the integration server 40, and each of the servers includes the communication unit 41, the data input and output unit 42, the server storage unit 43, and the processing unit 44. The communication unit 41, the data input and output unit 42, the server storage unit 43, and the processing unit 44 are communicably connected to each other.

In the present embodiment, the server storage unit 43 of the mobility server 40A includes the vehicle data storage unit 43a, the ID data storage unit 43b, the user data storage unit 43c, and the terminal data storage unit 43d in terms of functional concept. The server storage unit 43 of the medical server 40B includes a vehicle data storage unit 43a, an abnormality detection data storage unit 43e, and a medical information data storage unit 43f in terms of functional concept. The other basic configuration of the abnormality detection system 1 according to the present embodiment is the same as the basic configuration of the abnormality detection system 1 according to the first embodiment described above. The mobility server 40A and medical server 40B described above are typically configured as so-called relational servers that store various kinds of data in a table format such as CSV or Excel, but are not limited thereto. For example, the mobility server 40A and the medical server 40B may be configured as so-called NoSQL servers that store various kinds of data in a format such as XML or JSON. In this case, for example, the mobility server 40A and the medical server 40B can be configured to easily search for a large amount of data at the same time.

In the present embodiment, when determining the presence or absence of the abnormal driving of the user in the vehicle V from the vehicle data D1, the detection device 50 acquires the vehicle data D1 of a type necessary for determining the abnormal driving of the user from the vehicle data D1 stored in the mobility server 40A. Here, the mobility server 40A transmits the vehicle data D1 and the user data D2 in association with each other to the detection device 50. The detection device 50 detects the abnormal driving of the user based on the vehicle data D1 acquired from the mobility server 40A, and generates the abnormality detection data D4 including a content related to the detected abnormal driving. The detection device 50 transmits the abnormality detection data D4 in association with the vehicle identification information D10 of the vehicle data D1 to the medical server 40B. When an abnormal driving of the user is detected, the detection device 50 transmits the user data D2 and the abnormality detection data D4 in association with each other to the medical institution terminal 70.

In the medical institution, the medical worker such as a doctor utilizes the medical institution terminal 70 to create the medical information data D5 based on the abnormality detection data D4 and the opinion of the medical worker himself or herself. The medical institution terminal 70 transmits the medical information data D5 to the detection device 50. The detection device 50 transmits the medical information data D5 in association with the vehicle identification information D10 of the vehicle data D1 to the medical server 40B and stores the data in the storage unit. The detection device 50 specifies the user terminal 60 from the user data D2 corresponding to the medical information data D5, and issues the medical information notification including the content based on the medical information data D5 to the specified user terminal 60. Here, the detection device 50 transmits the medical information notification in association with the vehicle identification information D10 of the vehicle data D1 or the user data D2 to the mobility server 40A, and the mobility server 40A specifies the user terminal 60 by collating the user data D2 corresponding to the vehicle identification information D10 associated with the medical information notification or collating the user data D2 matching the user data D2 associated with the medical information notification, and transmits the medical information notification to the user terminal 60.

### <Flow of Data in Abnormality Detection System>

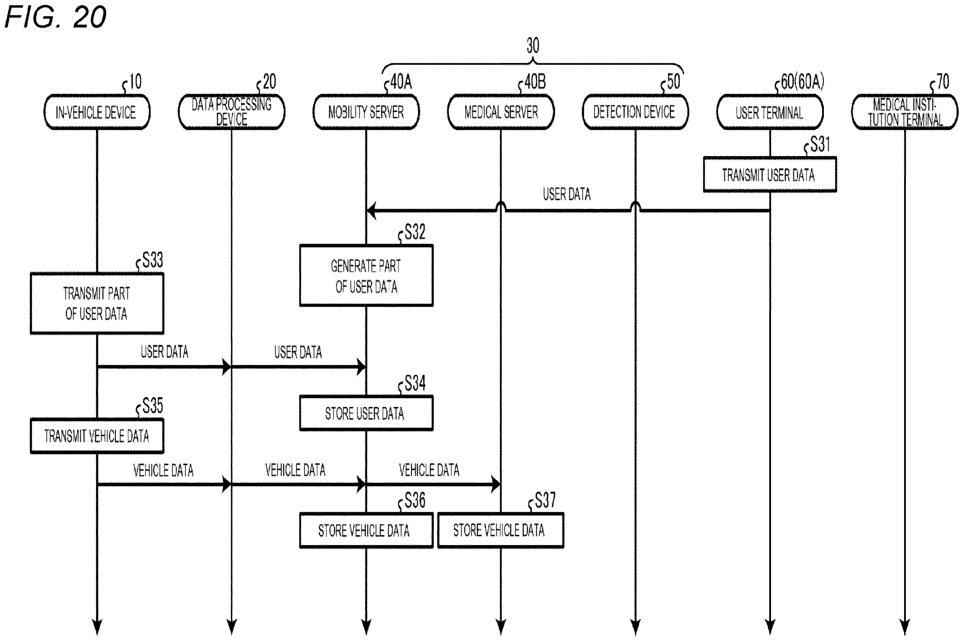

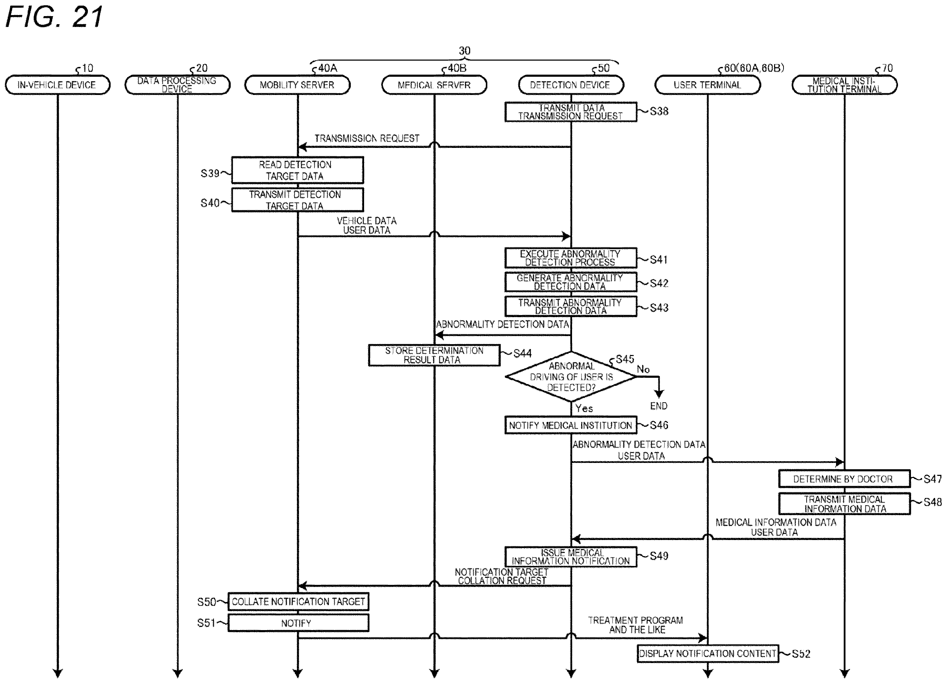

Next, an example of a flow of each process in the abnormality detection system 1 according to the present embodiment will be described with reference to FIGS. 20 and 21. Similarly to the description of the present embodiment, in the following description, the transmission and reception of data are performed via the network NW, but a detailed description of this point may be omitted. In the following description, in order to mainly focus on the flow of data, a description of specific processing operations related to the storage unit and the processing unit in each device may be omitted as appropriate, and an outline of the entire configuration described above will be referred to as necessary. The flow of each process described below is merely an example of a representative process, and the present invention is not limited thereto.

### <Storage of User Data and Vehicle Data>

First, as shown in FIG. 20, the user terminal 60 (the first user terminal 60A) transmits, as the user data D2 to the mobility server 40A, data related to the user basic information D20, the service setting information D23, the favorite information D25, and the like registered in the user terminal 60 (the first user terminal 60A) in the user data D2 (step S31), for example. The user terminal 60 (the first user terminal 60A) is capable of transmitting not only the initially registered user data D2 but also the user data D2 updated after the initial registration to the mobility server 40A. The user terminal 60 (the first user terminal 60A) can transmit, for example, the user basic information D20 including the terminal registration information of the second user terminal 60B as the user data D2 such that the notification of the consultation recommendation for the user is also issued to the second user terminal 60B mainly used by the family of the user, the related person, or the like.

In response to receiving the user data D2 from the user terminal 60 (the first user terminal 60A), the mobility server 40A automatically generates a part of the user data D2 based on the received user data D2 (step S32). Here, the mobility server 40A automatically generates data related to the user attribute information D21, the agreement history information D22, the contract package information D24, and the like based on data related to the user basic information D20, the service setting information D23, the favorite information D25, and the like received from the user terminal 60, for example. The mobility server 40A stores the generated user data D2 in the server storage unit 43 (the user data storage unit 43c).

The user data D2 may be registered, for example, via the in-vehicle device 10. In this case, the in-vehicle device 10 can register a part of the information of the user data D2 in accordance with an operation on the HMI unit 12 performed by the user, and also can transmit data related to the registered part of the information as the user data D2 to the data processing device 20, and transmit the data to the mobility server 40A via the data processing device 20 (step S33).

The mobility server 40A stores the received user data D2 and the automatically generated user data D2 in association with the user ID D60 in the server storage unit 43 (step S34).

Similarly to the first embodiment, the in-vehicle device 10 transmits the vehicle data D1 detected or acquired by the detection and acquisition unit 11 to the data processing device 20 at an appropriate timing (step S35). The in-vehicle device 10 transmits the vehicle data D1 to the data processing device 20 in a state where the vehicle identification information D10 and the corresponding vehicle data D1 are associated with each other. In response to receiving the vehicle data D1 from the in-vehicle device 10, the data processing device 20 transmits the received vehicle data D1 to the mobility server 40A.

In response to receiving the vehicle data D1 from the data processing device 20, the mobility server 40A stores the vehicle data D1 in association with the vehicle ID D61 in the server storage unit 43. In this case, since the user ID D60 and the vehicle ID D61 are associated with each other based on the ID data D6, the vehicle data D1 and the user data D2 can be associated and stored in the server storage unit 43 of the mobility server 40A (step S36). In the vehicle data D1, the vehicle data D1 of the type required for detecting the abnormal driving of the user is transmitted from the mobility server 40A to the medical server 40B, and stored in the server storage unit 43 (the vehicle data storage unit 43a) of the medical server 40B (step S37).

### <Detection of Abnormal Driving of User and Medical Information Notification>

As shown in FIG. 21, when the detection device 50 acquires, from the mobility server 40A, the vehicle data D1 and the user data D2 used for the detection of the abnormal driving of the user and the medical information notification, the detection device 50 transmits a data transmission request to the mobility server 40A (step S38). In response to receiving the data transmission request from the detection device 50, the mobility server 40A reads the target vehicle data D1 and the user data D2 corresponding to the vehicle data D1 (step S39). The mobility server 40A transmits the read vehicle data D1 and the user data D2 in association with each other to the detection device 50 (step S40). In response to receiving the vehicle data D1 and the user data D2, the detection device 50 detects the abnormal driving of the user in the vehicle V from the vehicle data D1 (step S41). Then, the detection device 50 generates the abnormality detection data D4 including the content of the detection result, and stores the abnormality detection data D4 in the detection storage unit 54 (step S42). Here, the detection device 50 stores the abnormality detection data D4 in the detection storage unit 54 in association with the vehicle data D1 and the user data D2. Thereafter, the detection device 50 transmits the generated abnormality detection data D4 to the medical server 40B (step S43). Here, when the detection device 50 transmits the abnormality detection data D4 to the medical server 40B, the medical server 40B collates the abnormality detection data D4 received from the detection device 50 with the vehicle data D1, the user data D2, or the like stored in the medical server 40B, and transmits the data in a manner of being able to store in association with each other. For example, the detection device 50 transmits the abnormality detection data D4 to the medical server 40B in association with the vehicle identification information D10 of the vehicle data D1 and the user basic information D20 of the user data D2. Then, the medical server 40B collates the abnormality detection data D4 received from the detection device 50 with the vehicle data D1, the user data D2, or the like stored in the medical server 40B, stores the data in the server storage unit 43 in association with each other, and manages the data (step S44).

The detection device 50 confirms whether the abnormal driving of the user is detected based on the abnormality detection data D4 (step S45). Here, if the detection device 50 confirms that there is no abnormality in driving of the user in the vehicle V based on the abnormality detection data D4, the process is ended until a next data transmission request is transmitted. If the detection device 50 confirms that there is an abnormality in driving of the user based on the abnormality detection data D4 (if the abnormal driving of the user is detected), the detection device 50 transmits the user data D2 and the abnormality detection data D4 in association with each other to the medical institution terminal 70 of the medical institution (step S46). In this case, similarly to the first embodiment described above, the detection device 50 may perform, with respect to the user, the agreement confirmation for transmitting the user data D2 and the abnormality detection data D4 to the medical institution terminal 70 of the medical institution. When performing the agreement confirmation, the detection device 50 specifies the user terminal 60 (the first user terminal 60A) of the user that performs the agreement confirmation based on the user data D2, and notifies the user terminal 60 (the first user terminal 60A) of the agreement confirmation. In response to an operation from the user, the user terminal 60 (the first user terminal 60A) transmits, to the detection device 50, a response indicating whether to agree to transmit the user data D2 and the abnormality detection data D4 to the medical institution terminal 70 of the medical institution. The detection device 50 transmits the user data D2 and the abnormality detection data D4 to the medical institution terminal 70 of the medical institution only when the user agrees to transmit the user data D2 and the abnormality detection data D4 to the medical institution terminal 70 of the medical institution.

In response to receiving the user data D2 and the abnormality detection data D4 transmitted from the detection device 50, the medical institution terminal 70 stores the user data D2 and the abnormality detection data D4 in association with each other in the medical institution storage unit 74 and manages the data. The medical institution terminal 70 processes the user data D2 and the abnormality detection data D4 to be utilizable by the medical worker such as a doctor in the medical institution. The processed user data D2 and the abnormality detection data D4 are utilized by the medical worker in the medical institution to determine the presence or absence of the indication or the symptom of the disease related to the cognitive function of the user. The medical worker in the medical institution determines the presence or absence of the indication or the symptom of the disease related to the cognitive function of the user based on the abnormality detection data D4, the opinion of the medical worker himself or herself, and the like (step S47). Then, the medical worker such as a doctor in the medical institution creates the medical information data D5 based on the determination result, and stores the medical information data D5 in the medical institution storage unit 74 of the medical institution terminal 70. Here, the medical institution terminal 70 stores the medical information data D5 in association with the user data D2 and the abnormality detection data D4 in the medical institution storage unit 74. The medical institution terminal 70 transmits the medical information data D5 to the detection device 50 (step S48). Here, when the medical institution terminal 70 transmits the medical information data D5 to the detection device 50, the detection device 50 collates the medical information data D5 received from the medical institution terminal 70 with the user data D2 or the like stored in the detection device 50, and transmits the data in a manner of being able to be stored in association with each other. Specifically, for example, the medical institution terminal 70 transmits the medical information data D5 to the detection device 50 in association with the user data D2 corresponding to the medical information data D5. Then, the detection device 50 collates the medical information data D5 received from the medical institution terminal 70 with the user data D2 or the like stored in the detection device 50, stores the data in the detection storage unit 54 in association with each other, and manages the data.

The detection device 50 transmits the medical information including the content based on the medical information data D5 to the mobility server 40A and issues A medical information notification (step S49). In response to receiving the medical information from the detection device 50, the mobility server 40A performs collation on a target to be notified of the medical information (step S50). By collating the notification target, the user terminal 60 of the user to be notified of the medical information is specified. Here, when issuing the medical information notification to the user terminal 60 via the mobility server 40A, the detection device 50 transmits, to the mobility server 40A, the medical information including the treatment program and the like based on the medical information data D5 in a state in which the mobility server 40A can specify the user terminal 60 to which the medical information notification is issued. For example, the detection device 50 transmits the user data D2 and the medical information including the treatment program and the like in association with each other to the mobility server 40A, so that the mobility server 40A can specify the user terminal 60 of the user to be notified of the medical information based on the user data D2 received from the detection device 50. For example, the detection device 50 transmits the vehicle identification information D10 included in the vehicle data D1 and the medical information including the treatment program and the like to the mobility server 40A in association with each other, so that the mobility server 40A can specify the user terminal 60 of the user to be notified of the medical information based on the vehicle identification information D 10 received from the detection device 50.

When specifying the user terminal 60 to be notified of the medical information, the mobility server 40A transmits the medical information notification to the specified user terminal 60 (step S51). In response to receiving the medical information notification from the mobility server 40A, the user terminal 60 displays the content corresponding to the medical information notification on the display unit (step S52). For example, when the content of the medical information notification is a determination result related to the cognitive function of the user, information related to the presence or absence, the content, and the like of the indication or the symptom of the disease related to the cognitive function of the user is output to the user terminal 60. When the content of the medical information notification includes the treatment program, information related to the treatment program determined to be suitable for the user by the doctor of the medical institution is output to the user terminal 60. When the content of the medical information notification includes the consultation recommendation on the disease related to the cognitive function, the information related to the consultation recommendation as shown in FIGS. 10 to 12 is output.

### <Effects of Second Embodiment>

As described above, in the abnormality detection system 1 according to the second embodiment, the service providing device 30 includes the mobility server 40A that manages the user data D2 and the vehicle data D 1, the medical server 40B that manages the abnormality detection data D4, and the detection device 50 that communicates with the medical server 40B, the mobility server 40A and the medical server 40B constitute a storage unit in cooperation with each other, and manage the user data D2, the vehicle data D 1, and the abnormality detection data D4 in association with each other, and the detection device 50 includes the detection unit and the notification unit.

The abnormality detection system 1 according to the present embodiment is configured to manage the vehicle data D1 and the user data D2 by the mobility server 40A, and manage the abnormality detection data D4 including the information having high confidentiality and related to the body of the user by the medical server 40B different from the mobility server 40A. According to this configuration, the user data D2 including personal information and the abnormality detection data D4 having high confidentiality are managed separately so as not to be directly associated with each other, and thus, a secret retention can be improved and various kinds of data can be smoothly utilized. The abnormality detection system 1 according to the present embodiment can be used for, in addition to the medical information notification issued by the service providing device 30, various services and analyses by enabling the vehicle data D 1 managed by a mobility service providing device 30A to be utilized by an agency (dealer) or a developer of the vehicle V. For example, the mobility service providing device 30A is utilized for a service related to maintenance of the vehicle V In addition, in the abnormality detection system 1 according to the present embodiment, since the server is divided according to the use, it is possible to acquire the vehicle data D1 in the data type or the suitable cycle according to the use. Accordingly, the server can be maintained without affecting each other, and the storage resource can be effectively used.

In the abnormality detection system 1 according to the second embodiment, the vehicle data D 1 includes the vehicle identification information D 10 for identifying the vehicle, the mobility server 40A stores the user data D2 and the vehicle identification information D10 in association with each other, and the medical server 40B stores the abnormality detection data D4 and the vehicle identification information D 10 in association with each other.

By associating the user data D2 with the abnormality detection data D4 via the vehicle identification information D10, the abnormality detection system 1 according to the present embodiment can manage the user data D2 of the mobility server 40A and the abnormality detection data D4 of the medical server 40B and the like on different servers without directly correlating the data, and confidentiality of data management can be improved.

### <Third Embodiment>

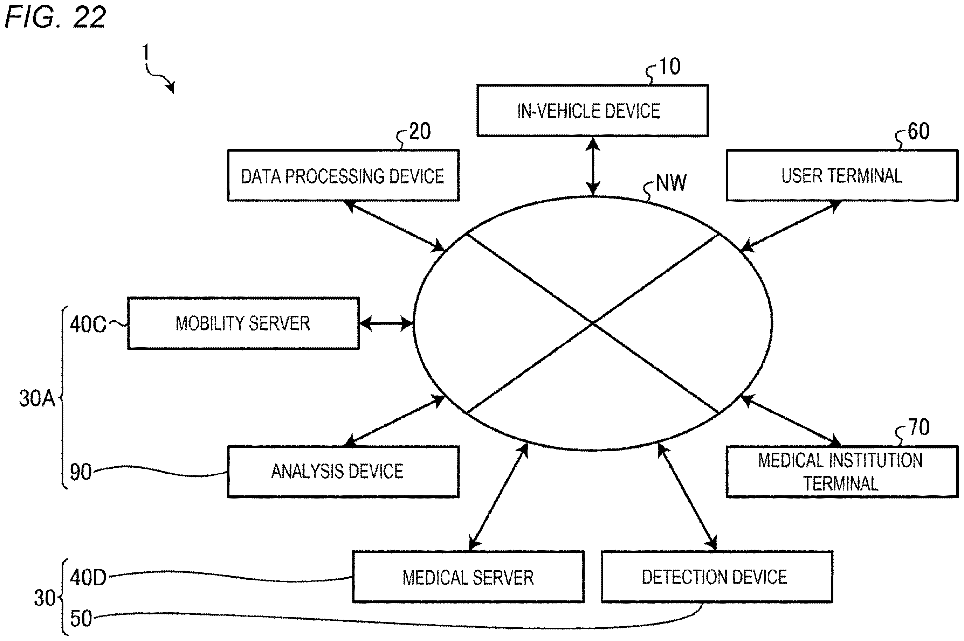

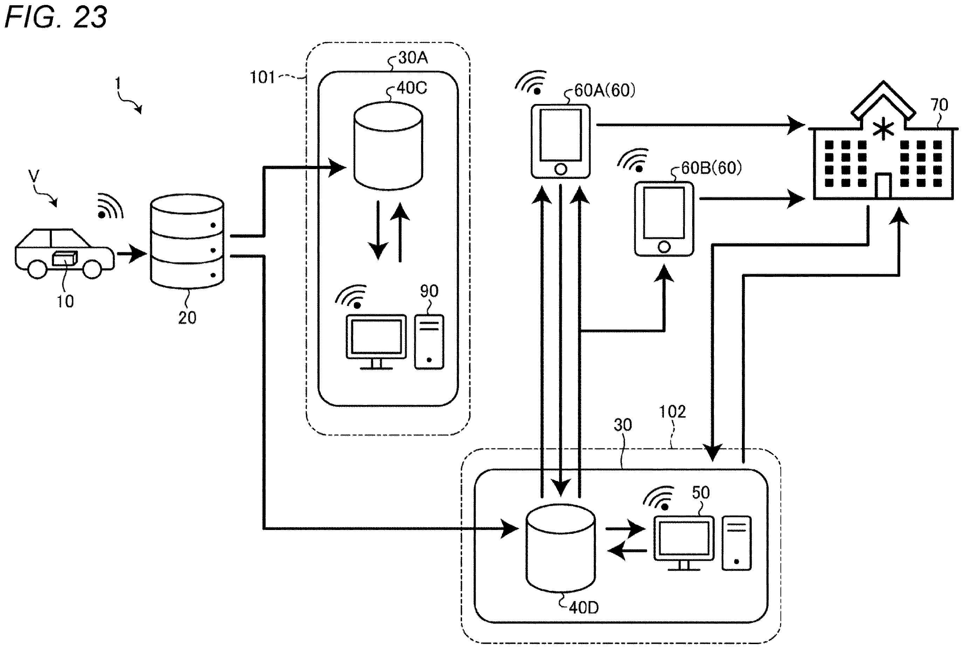

Next, a third embodiment will be described with reference to FIGS. 22 to 28. As shown in FIGS. 22 and 23, the abnormality detection system 1 and the base station device (the service providing device 30) according to the present embodiment are different from the abnormality detection system 1 and the base station device (the service providing device 30) according to the first embodiment described above in that the mobility service providing device 30A including a mobility server 40C and an analysis device 90 is provided. In the abnormality detection system 1 according to the present embodiment, the service providing device 30 includes a medical server 40D and the detection device 50. The mobility service providing device 30A has a function different from that of the service providing device 30 and constitutes a system capable of managing various kinds of data related to the vehicle V independently of the service providing device 30 and using the data for various services and analyses. The mobility server 40C and the medical server 40D are configured not to cooperate with each other, and the detection device 50 cannot access the mobility server 40C, and the analysis device 90 cannot access the medical server 40D. In the present embodiment, the mobility service providing device 30A is communicably connected to other terminal devices (not shown), for example, a terminal device of an agency (dealer) or a developer of the vehicle V via the network NW. For example, the mobility service providing device 30A can receive the vehicle data D1 from the vehicle V and use the vehicle data D1 for various services and analyses by allowing the vehicle data D1 to be used by an agency (dealer) or a developer of the vehicle V.

### <Basic Configuration of Mobility Service Providing Device>

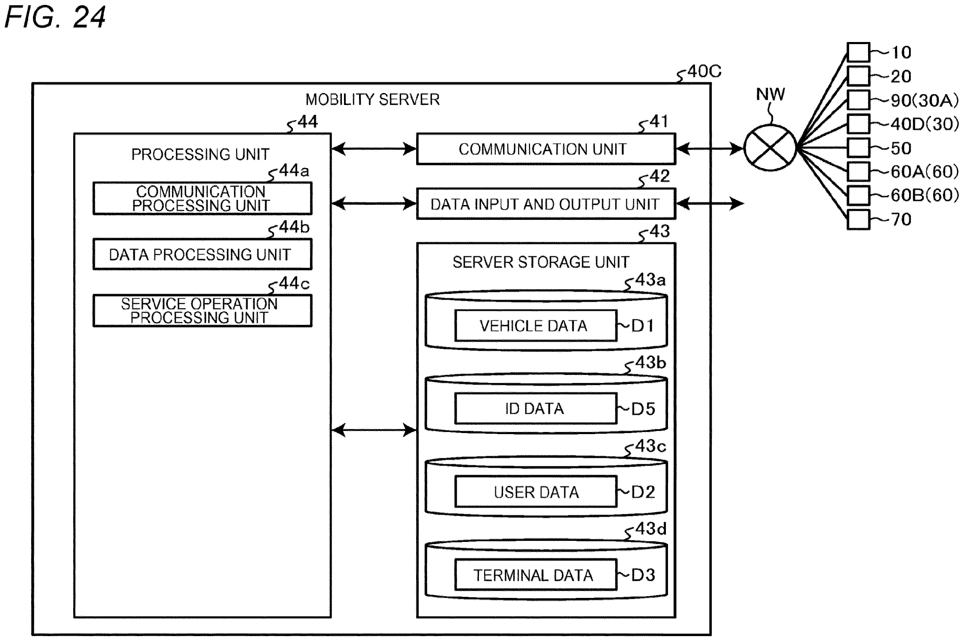

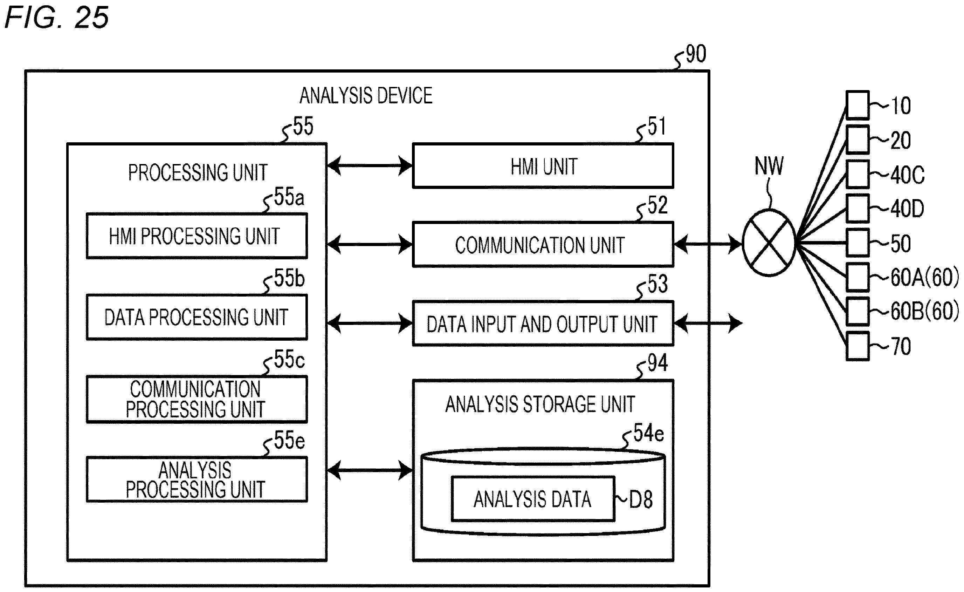

As described above, the mobility service providing device 30A includes the mobility server 40C and the analysis device 90. As shown in FIG. 24, a basic configuration of the mobility server 40C according to the present embodiment is substantially the same as that of the mobility server 40A shown in FIG. 18. As shown in FIG. 25, a basic configuration of the analysis device 90 is substantially the same as that of the detection device 50 shown in FIG. 8, but is different from the detection device 50 shown in FIG. 8 in that an analysis storage unit 94 is provided instead of the detection storage unit 54 and an analysis processing unit 55e is provided instead of the detection processing unit 55d in terms of functional concept. The analysis storage unit 94 includes an analysis data storage unit 54e in terms of functional concept. The analysis data storage unit 54e is a storage area for storing analysis data D8.

The analysis device 90 is a device that performs various analyses (quality investigation, accident analysis, failure analysis, feedback to development, mobility planning, utilization to new business, and the like) based on data stored in the mobility server 40C. The analysis device 90 is typically a device used by a developer or an analysis business operator of the vehicle V.

The analysis processing unit 55e is a unit having a function capable of executing a process of analyzing data stored in the mobility server 40C in accordance with an operation on the operation input device constituting the HMI unit 51. The analysis processing unit 55e executes a process of acquiring the vehicle data D1 from the mobility server 40C via the communication unit 52 in accordance with an operation on the operation input device constituting the HMI unit 51. In response to receiving data necessary for executing the requested analysis in the analysis device 90 from the vehicle data D1 and the like stored in the mobility server 40C, the analysis processing unit 55e analyzes the data based on the data.

The data processing unit 55b of the analysis device 90 is capable of executing a process of storing an analysis result obtained by the analysis processing unit 55e as the analysis data D8 in the analysis data storage unit 54e. The HMI processing unit 55a of the analysis device 90 is capable of executing a process of controlling an information output device constituting the HMI unit 51 and outputting the analysis data D8 such as the analysis result obtained by the analysis processing unit 55e via the information output device.

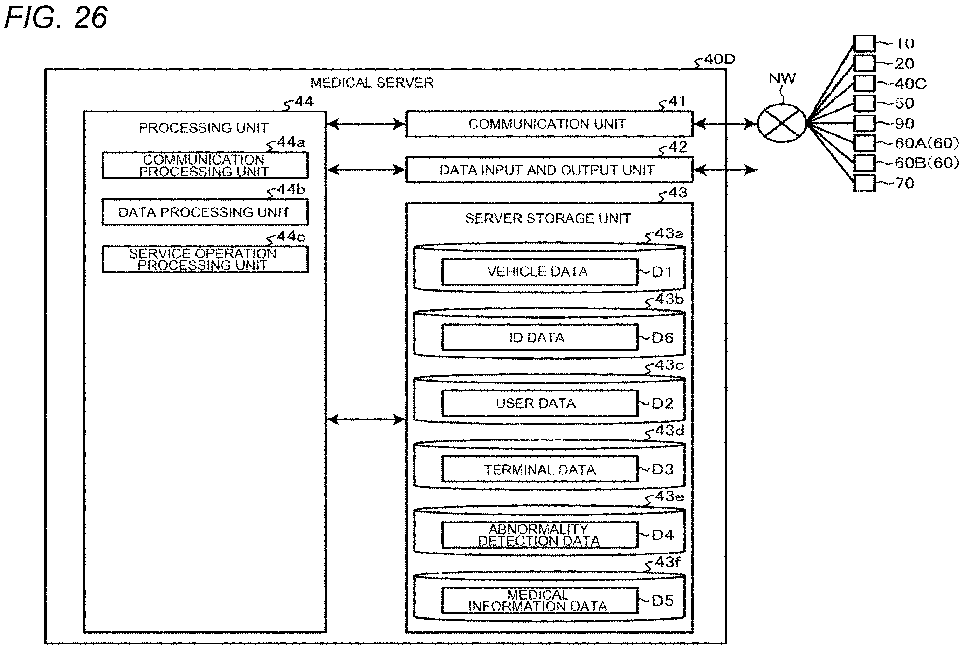

In the present embodiment, the mobility service providing device 30A constitutes a first base station device disposed in the mobility base station 101, and the service providing device 30 is disposed in the medical base station 102 and constitutes a second base station device. The mobility base station 101 is a base station that relays communication between the vehicle V and other terminal devices (a terminal device of an agency (dealer) or a developer of the vehicle V), and is, for example, a base station operated by a vehicle manufacturer. The medical base station 102 is a base station that relays communication among the vehicle V, the medical institution terminal 70, and the user terminal 60, and is, for example, a base station operated by a business operator who performs a service related to medical care. The analysis device 90 may access the second base station device from a remote location different from a location of the mobility base station 101. That is, the second base station device may include at least the medical server 40D. The analysis device 90 does not need to be disposed in the mobility base station 101, and may be managed in a place different from the location of the mobility base station 101. For example, the analysis device 90 may access the base station device from a remote location different from the location of the mobility base station 101. That is, the first base station device may include at least the mobility server 40C. As shown in FIG. 26, a basic configuration of the medical server 40D of the service providing device 30 is substantially the same as that of the integration server 40 according to the first embodiment shown in FIG. 6.

### <Flow of Data in Abnormality Detection System>

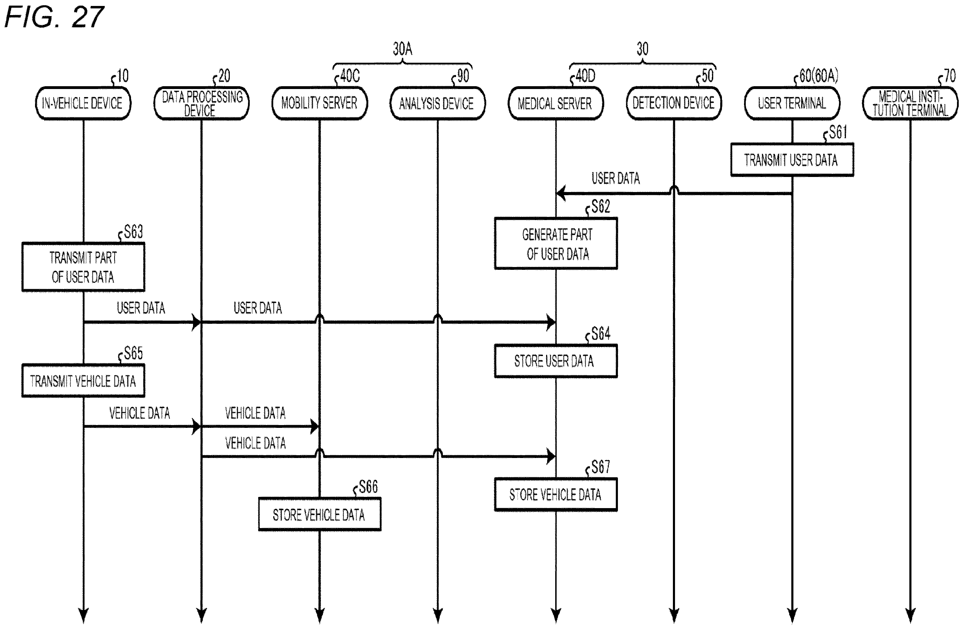

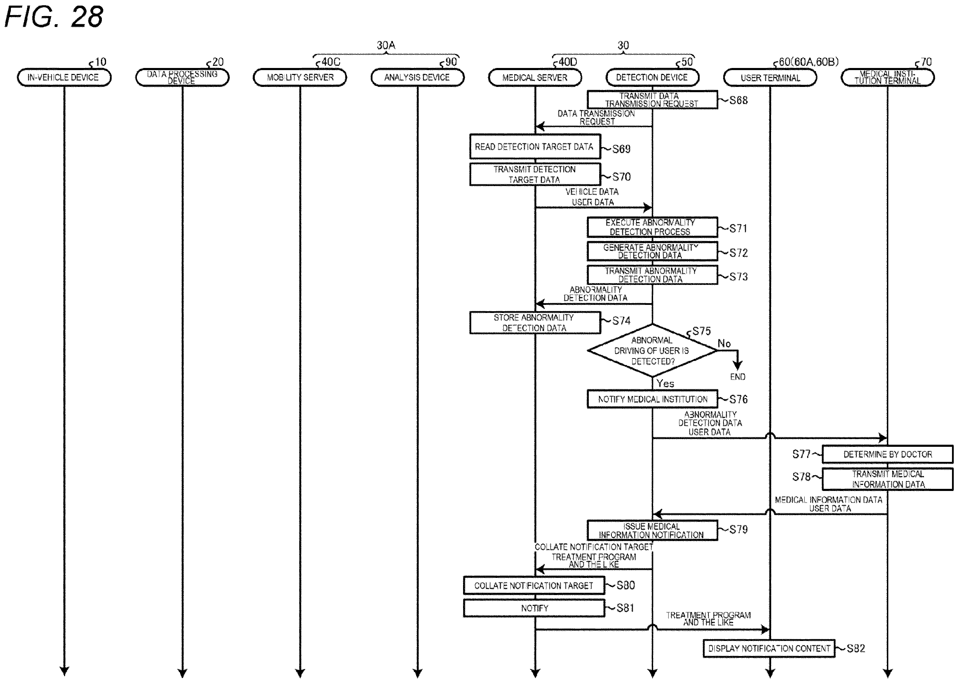

Next, an example of a flow of each process in the abnormality detection system 1 according to the present embodiment will be described with reference to FIGS. 27 and 28. In the following description, similarly to the descriptions of the first and second embodiments, transmission and reception of data are performed via the network NW, but a detailed description of this point may be omitted. In the following description, in order to mainly focus on the flow of data, a description of specific processing operations related to the processing unit and the like in each device may be omitted as appropriate, and the outline of the entire configuration described above will be referred to as necessary. The flow of each process described below is merely an example of a representative process, and the present invention is not limited thereto.

### <Storage of User Data and Vehicle Data>

First, as shown in FIG. 27, the user terminal 60 (the first user terminal 60A) transmits, as the user data D2 to the medical server 40D, data related to the user basic information D20, the service setting information D23, the favorite information D25, and the like registered in the user terminal 60 (the first user terminal 60A) in the user data D2 (step S61). The user terminal 60 (the first user terminal 60A) is capable of transmitting not only the initially registered user data D2 but also the user data D2 updated after the initial registration to the medical server 40D. The user terminal 60 (the first user terminal 60A) can transmit, for example, the user basic information D20 including the terminal registration information of the second user terminal 60B as the user data D2 such that the notification of the consultation recommendation for the user is also issued to the second user terminal 60B mainly used by the family of the user, the related person, or the like.

In response to receiving the user data D2 from the user terminal 60 (the first user terminal 60A), the medical server 40D automatically generates a part of the user data D2 based on the received user data D2 (step S62). Here, the medical server 40D automatically generates data related to the user attribute information D21, the agreement history information D22, the contract package information D24, and the like based on data related to the user basic information D20, the service setting information D23, the favorite information D25, and the like received from the user terminal 60, for example.

The user data D2 may be registered, for example, via the in-vehicle device 10. In this case, the in-vehicle device 10 can also register a part of the information of the user data D2 in accordance with an operation on the HMI unit 12 performed by the user, transmit data related to the registered part of the information as the user data D2 to the data processing device 20, and transmit the data to the medical server 40D via the data processing device 20 (step S63).

The medical server 40D stores the received user data D2 and the automatically generated user data D2 in the server storage unit 43 (the user data storage unit 43c) in association with the user ID D60 (step S64).

The in-vehicle device 10 transmits the vehicle data D1 detected and acquired by the detection and acquisition unit 11 to the data processing device 20 at an appropriate timing (step S65). Here, the vehicle data D1 includes the vehicle identification information D10. In response to receiving the vehicle data D1 from the in-vehicle device 10, the data processing device 20 transmits the received vehicle data D1 to the medical server 40D and the mobility server 40C.

In response to receiving the vehicle data D1 from the data processing device 20, the mobility server 40C stores the received vehicle data D1 in the server storage unit 43 (the vehicle data storage unit 43a) (step S66), and makes the data available for various services. In response to receiving the vehicle data D1 from the data processing device 20, the medical server 40D stores the vehicle data D 1 in the server storage unit 43 (vehicle data storage unit 43a) in association with the vehicle ID D61. In this case, since the user ID D60 and the vehicle ID D61 are associated with each other based on the ID data D6, the vehicle data D1 and the user data D2 can be associated and stored in the server storage unit 43 (the vehicle data storage unit 43a) (step S67).

### <Detection of Abnormal Driving of User and Medical Information Notification>

As shown in FIG. 28, in response to acquiring the vehicle data D1 used to detect the abnormal driving of the user from the medical server 40D, the detection device 50 transmits a data transmission request to the medical server 40D (step S68). In response to receiving the data transmission request from the detection device 50, the medical server 40D reads the target vehicle data D1 and the user data D2 corresponding to the vehicle data D1 (step S69). Then, the medical server 40D transmits the read vehicle data D1 and the user data D2 corresponding to the vehicle data D1 to the detection device 50 (step S70). Here, the medical server 40D transmits the vehicle data D1 and the user data D2 in association with each other to the detection device 50. In response to receiving the vehicle data D1 and the user data D2, the detection device 50 detects the abnormal driving of the user in the vehicle V from the vehicle data D1 (step S71). Then, the detection device 50 generates the abnormality detection data D4 including the content of the detection result, and stores the abnormality detection data D4 in the detection storage unit 54 (step S72). Here, the detection device 50 stores the abnormality detection data D4 in the detection storage unit 54 in association with the vehicle data D1 and the user data D2. Thereafter, the detection device 50 transmits the generated abnormality detection data D4 to the medical server 40D (step S73). Here, when the detection device 50 transmits the abnormality detection data D4 to the medical server 40D, the medical server 40D collates the abnormality detection data D4 received from the detection device 50 with the user data D2 or the like stored in the medical server 40D, and transmits the data in a manner of being able to store in association with each other. Specifically, for example, the detection device 50 transmits the abnormality detection data D4 to the medical server 40D in association with the vehicle data D1 and the user data D2. Then, the medical server 40D collates the abnormality detection data D4 received from the detection device 50 based on the vehicle identification information D10 included in the vehicle data D1, the user data D2, or the like stored in the medical server 40D, stores the data in the server storage unit 43 in association with each other, and manages the data (step S74).

The detection device 50 confirms whether the abnormal driving of the user is detected based on the abnormality detection data D4 (step S75). Here, if the detection device 50 confirms that there is no abnormality in driving of the user in the vehicle V based on the abnormality detection data D4, the process is ended until a next data transmission request is transmitted. If the detection device 50 confirms that there is an abnormality in driving of the user based on the abnormality detection data D4 (if the abnormal driving of the user is detected), the detection device 50 transmits the user data D2 and the abnormality detection data D4 in association with each other to the medical institution terminal 70 of the medical institution (step S76). In this case, the detection device 50 may perform, with respect to the user, an agreement confirmation for transmitting the user data D2 and the abnormality detection data D4 to the medical institution terminal 70 of the medical institution. When performing the agreement confirmation, the detection device 50 specifies the user terminal 60 (the first user terminal 60A) of the user that performs the agreement confirmation based on the user data D2, and notifies the user terminal 60 (the first user terminal 60A) of the agreement confirmation. In response to an operation from the user, the user terminal 60 (the first user terminal 60A) transmits, to the detection device 50, a response indicating whether to agree to transmit the user data D2 and the abnormality detection data D4 to the medical institution terminal 70 of the medical institution. The detection device 50 transmits the user data D2 and the abnormality detection data D4 to the medical institution terminal 70 of the medical institution only when the user agrees to transmit the user data D2 and the abnormality detection data D4 to the medical institution terminal 70 of the medical institution.

In response to receiving the user data D2 and the abnormality detection data D4 transmitted from the detection device 50, the medical institution terminal 70 stores the user data D2 and the abnormality detection data D4 in association with each other in the medical institution storage unit 74 and manages the data. The medical institution terminal 70 processes the user data D2 and the abnormality detection data D4 to be utilizable by the medical worker such as a doctor in the medical institution. The processed user data D2 and the abnormality detection data D4 are utilized by a doctor in the medical institution to determine the presence or absence of the indication or the symptom of the disease related to the cognitive function of the user. The medical worker in the medical institution determines the presence or absence of the indication or the symptom of the disease related to the cognitive function of the user based on the abnormality detection data D4, the opinion of the medical worker himself or herself, and the like (step S77). Then, the medical worker such as a doctor in the medical institution creates the medical information data D5 based on the determination result, and stores the medical information data D5 in the medical institution storage unit 74 of the medical institution terminal 70. Here, the medical institution terminal 70 stores the medical information data D5 in association with the user data D2 and the abnormality detection data D4 in the medical institution storage unit 74. The medical institution terminal 70 transmits the medical information data D5 to the detection device 50 (step S78). Here, when the medical institution terminal 70 transmits the medical information data D5 to the detection device 50, the detection device 50 collates the medical information data D5 received from the medical institution terminal 70 with the user data D2 or the like stored in the detection device 50, and transmits the data in a manner of being able to be stored in association with each other. Specifically, for example, the medical institution terminal 70 transmits the medical information data D5 to the detection device 50 in association with the user data D2 corresponding to the medical information data D5. Then, the detection device 50 collates the medical information data D5 received from the medical institution terminal 70 with the user data D2 or the like stored in the detection device 50, stores the data in the detection storage unit 54 in association with each other, and manages the data. The detection device 50 transmits the medical information including the content based on the medical information data D5 to the medical server 40D and issues a medical information notification (step S79).

In response to receiving the medical information from the detection device 50, the medical server 40D performs collation on a target to be notified of the medical information (step S80). By collating the notification target, the user terminal 60 of the user to be notified of the medical information is specified. Here, when issuing the medical information notification to the user terminal 60 via the medical server 40D, the detection device 50 transmits, to the medical server 40D, the medical information including the treatment program and the like based on the medical information data D5 in a state in which the medical server 40D can specify the user terminal 60 to which the medical information notification is issued. For example, the detection device 50 transmits the user data D2 and the medical information including the treatment program and the like in association with each other to the medical server 40D, so that the medical server 40D can specify the user terminal 60 of the user to be notified of the medical information based on the user data D2 received from the detection device 50. For example, the detection device 50 transmits the vehicle identification information D10 included in the vehicle data D1 and the medical information including the treatment program and the like to the mobility server 40C in association with each other, so that the mobility server 40C can specify the user terminal 60 of the user to be notified of the medical information based on the vehicle identification information D10 received from the detection device 50.

When specifying the user terminal 60 to be notified of the medical information, the medical server 40D transmits the medical information notification to the specified user terminal 60 (step S81). In response to receiving the medical information notification from the medical server 40D, the user terminal 60 displays the content corresponding to the medical information notification on the display unit (step S82). For example, when the content of the medical information notification is a determination result related to the cognitive function of the user, information related to the presence or absence, the content, and the like of the indication or the symptom of the disease related to the cognitive function of the user is output to the user terminal 60. When the content of the medical information notification includes the treatment program, information related to the treatment program determined to be suitable for the user by the doctor of the medical institution is output to the user terminal 60. When the content of the medical information notification includes the consultation recommendation on the disease related to the cognitive function, the information related to the consultation recommendation as shown in FIGS. 10 to 12 is output.

### <Effects of Third Embodiment>

As described above, the abnormality detection system 1 according to the present embodiment further includes the mobility server 40C that manages the user data D2 related to the user of the vehicle V and the vehicle data D1 including the traveling information D13 of the vehicle V, the service providing device 30 includes the medical server 40D that is configured not to cooperate with the mobility server 40C and manages at least the abnormality detection data D4, and the detection device 50 configured to communicate with the medical server 40D, and the detection device 50 includes the detection unit and the notification unit.

In the abnormality detection system 1 according to the present embodiment, the abnormality detection data D4 including information having high confidentiality and related to the body of the user is managed by the medical server 40D, and the user data D2 and the vehicle data D1 utilized in the mobility service are managed separately by the mobility server 40C different from the medical server 40D, so that it is possible to smoothly utilize data used for mobility services while improving secret retention. For example, in the abnormality detection system 1 according to the present embodiment, in addition to the notification of the medical information by the service providing device 30, the vehicle data D1 can also be used by an agency (dealer) or a developer of the vehicle V by the mobility service providing device 30A, and thereby the data can be utilized for various services and analyses. Since the mobility server 40C and the medical server 40D are provided separately and configured not to cooperate with each other, the user data D2 managed by the mobility server 40C and the abnormality detection data D4 managed by the medical server 40D are not directly managed in association with each other, and the secret retention of personal information can be improved.

In the abnormality detection system 1 according to the present embodiment, since the detection device 50 cannot access the mobility server 40C and the analysis device 90 cannot access the medical server 40D, the detection device 50 cannot acquire or browse the user data D2 managed by the mobility server 40C, and the analysis device 90 cannot acquire or browse the abnormality detection data D4 managed by the medical server 40D, and thus the secret retention of personal information can be improved.

In addition, in the abnormality detection system 1 according to the present embodiment, since the server is divided according to the use, it is possible to acquire the vehicle data D1 in the data type or the suitable cycle according to the use. Accordingly, the server can be maintained without affecting each other, and the storage resource can be effectively used.

In the abnormality detection system 1 according to the present embodiment, the mobility server 40C is disposed in the mobility base station 101 that relays communication between the vehicle V and the user terminals 60, 60A, and 60B, and the medical server 40D is disposed in the medical base station 102 that relays communication between the vehicle V and the medical institution terminal 70 and is a base station different from the mobility base station 101. In this case, the user terminals 60, 60A, and 60B may not associate the service provided from the mobility server 40C with the service provided from the medical server 40D.

In the abnormality detection system 1 according to the present embodiment, in addition to the mobility server 40C, by managing the medical server 40D storing the abnormality detection data D4 and the like by a medical independent base station (medical base station), it is possible to improve the confidentiality of information, and to prevent data from being mixed and treated between the mobility server 40C and the medical server 40D.

The abnormality detection system 1 and the base station device according to each embodiment of the present invention are not limited to each embodiment described above, and various modifications can be made within the scope described in the claims.

### <Modification>

In the above description, the abnormality detection system 1 transmits the abnormality detection data D4 to the medical institution terminal 70 of the medical institution only when the abnormal driving of the user is detected, but the present invention is not limited thereto. For example, regardless of whether the abnormal driving of the user is detected, the abnormality detection data D4 may be periodically transmitted to the medical institution terminal 70, and a medical worker such as a doctor of the medical institution can confirm a change in a driving behavior of the user over a long period of time. According to this configuration, for example, the medical worker such as a doctor of the medical institution can confirm an indication or a symptom of a disease related to the cognitive function of the user, including no problem with the daily driving behavior of the user. If the medical worker of the medical institution can know not only the state when the abnormal driving of the user is detected, but also the state when there is no problem with the daily driving behavior of the user, the medical worker can immediately detect a change in the driving behavior of the user and take behavior to determine the indication or the symptom of the disease related to the cognitive function of the user.

In the above description, the abnormality detection system 1 collates the abnormality detection data D4 and the medical information notification corresponding to the vehicle identification information D10 between two different servers based on the vehicle identification information D10, but the present invention is not limited thereto. For example, the abnormality detection system 1 may collate the abnormality detection data D4 or the medical information notification based on an ID of the in-vehicle device 10, a manufacturing number of the in-vehicle device 10, a registrant number of the service in the abnormality detection system 1, or the like.

In the above description, the abnormality detection system 1 is configured such that the devices constituting the system transmit and receive data to and from each other through communication, but the present invention is not limited thereto. For example, a part or all of the data treated in the abnormality detection system 1 may be written to or read from a recording medium via the data input and output units 14, 22, 42, 53, 63, and 73 to transfer the data. Specifically, the vehicle data D1 collected by the in-vehicle device 10 may be written to a recording medium by the data input and output unit 14, read from the recording medium by the data input and output unit 22, and transferred to the data processing device 20. In the data processing device 20, the vehicle data D1 stored in the integration server 40 or the like by a worker may be written to the recording medium by the data input and output unit 22, and in the service providing device 30, the vehicle data D1 may be read from the recording medium by the data input and output unit 42 of the integration server 40 or the like, so that the vehicle data D1 is collectively stored in the integration server 40 or the like from the data processing device 20.

In the above description, the vehicle V may be a private vehicle or the like owned or used by the user, or may be a rental car, a shared car, or the like shared by unspecified utilizers. In a case where the vehicle V is a vehicle shared by unspecified utilizers, when the utilizer reserves or registers the use of the shared vehicle V, the abnormality detection system 1 executes a process of associating the user ID D60 with the vehicle ID D61 of the shared vehicle V from the in-vehicle device 10 or the user terminal 60 to the integration server 40 (or mobility server 40A).

Each of the processing units 16, 24, 44, 55, 65, and 75 described above may be implemented by a single processor, and the process related to each function may be executed, or may be implemented by a combination of a plurality of independent processors, and the process related to each function may be executed by each processor executing a program. The processes executed by the processing units 16, 24, 44, 55, 65, and 75 may be integrated into a single processing circuit or may be distributed to a plurality of processing circuits.

Each of the mobility server 40C and the analysis device 90 described above may be implemented by a single processor, and the process related to each function may be executed, or may be implemented by a combination of a plurality of independent processors, and the process related to each function may be executed by each processor executing a program. The processes executed by the mobility server 40C and the analysis device 90 may be integrated into a single processing circuit or may be distributed to a plurality of processing circuits.

Each of the medical server 40D and the detection device 50 described above may be implemented by a single processor, and the process related to each function may be executed, or may be implemented by a combination of a plurality of independent processors, and the process related to each function may be executed by each processor executing a program. The processes executed by the medical server 40D and the detection device 50 may be integrated into a single processing circuit or may be distributed to the plurality of processing circuits.

The abnormality detection system and the base station device according to the present embodiment may be configured by appropriately combining the components of the embodiments and the modifications described above.

### REFERENCE SIGNS LIST

- 1: abnormality detection system
- 10: in-vehicle device
- 20: data processing device
- 30: service providing device
- 40: integration server
- 40A, 40C: mobility server
- 40B, 40D: medical server
- 50: detection device
- 60: user terminal
- 70: medical institution terminal
- NW: network
- V: vehicle

## Claims

1. An abnormality detection system (1), comprising:
a service providing device (30) including a detection unit configured to detect abnormal driving of a user of a vehicle (V) from vehicle data (D1) including at least traveling information (D13) of the vehicle (V), and a notification unit configured to issue a notification related to medical information to the user; and
a user terminal (60, 60A, 60B) configured to communicate with the service providing device (30), wherein
the notification unit notifies the user terminal (60, 60A, 60B) of medical information created based on information related to the abnormal driving detected by the detection unit and an opinion of a medical worker of a medical institution.

2. The abnormality detection system according to claim 1, wherein
the service providing device further includes a storage unit configured to store user data related to the user of the vehicle and the vehicle data,
the detection unit stores abnormality detection data including a content of the detected abnormal driving in the storage unit,
the notification unit transmits at least the abnormality detection data to a medical institution terminal of the medical institution, receives, from the medical institution terminal, medical information data created based on the abnormality detection data and the opinion, and notifies the user terminal of the medical information including a content based on the medical information data.

3. The abnormality detection system according to claim 2, wherein
the medical information data includes information related to at least one of a treatment program determined to be suitable for the user by the medical worker, a determination result related to a cognitive function of the user, and a consultation recommendation on a disease related to the cognitive function.

4. The abnormality detection system according to claim 3, wherein
in a case where the medical information data includes the information related to the consultation recommendation,
the notification unit transmits the medical information including presentation of a behavior recommended to the user to the user terminal, and
the user terminal presents the behavior recommended to the user and displays an operation of executing the behavior in a receivable manner.

5. The abnormality detection system according to any one of claims 2 to 4, wherein
the storage unit stores the user data, the vehicle data, and the abnormality detection data in association with each other for each user, and
the user data includes information that can be collated with diagnosis information of a patient stored at the medical institution.

6. The abnormality detection system according to any one of claims 2 to 4, wherein
the service providing device includes an integration server configured to manage the user data, the vehicle data, and the abnormality detection data, and a detection device configured to communicate with the integration server,
the integration server includes the storage unit, and
the detection device includes the detection unit and the notification unit.

7. The abnormality detection system according to any one of claims 2 to 4, wherein
the service providing device includes a mobility server configured to manage the user data and the vehicle data, a medical server configured to manage the abnormality detection data, and a detection device configured to communicate with the medical server,
the mobility server and the medical server constitute the storage unit in cooperation with each other, and manage the user data, the vehicle data, and the abnormality detection data in association with each other, and
the detection device includes the detection unit and the notification unit.

8. The abnormality detection system according to any one of claims 2 to 4, further comprising:
a mobility server configured to manage the user data related to the user of the vehicle and the vehicle data including the traveling information of the vehicle, wherein
the service providing device includes a medical server configured not to cooperate with the mobility server and configured to manage the abnormality detection data at least, and a detection device configured to communicate with the medical server, and
the detection device includes the detection unit and the notification unit.

9. Abase station device (30), comprising:
a service providing device (30) including a storage unit configured to store user data (D2) related to a user of a vehicle (V) and vehicle data (D1) including traveling information (D13) of the vehicle (V), a detection unit configured to detect abnormal driving of the user in the vehicle from the vehicle data (D1) and store abnormality detection data (D4) including a content of the detected abnormal driving in the storage unit, and a notification unit configured to issue a notification related to medical information to the user, wherein
the notification unit transmits at least the abnormality detection data (D4) to a medical institution terminal (70) of a medical institution, receives, from the medical institution terminal (70), medical information data (D5) created based on the abnormality detection data (D4) and an opinion of a medical worker of the medical institution, and notifies a user terminal (60, 60A, 60B) of the medical information including a content based on the medical information data (D5).
